(19) Europäisches Patentamt
European Patent Office
Office européen des brevets

(11) EP 1 951 725 B1

(12) # EUROPEAN PATENT SPECIFICATION

(45) Date of publication and mention of the grant of the patent:
**03.11.2010 Bulletin 2010/44**

(21) Application number: **06829983.3**

(22) Date of filing: **14.11.2006**

(51) Int Cl.:
*C07D 491/10* (2006.01) *C07D 491/20* (2006.01)
*A61K 31/438* (2006.01) *A61P 27/06* (2006.01)
*A61P 25/00* (2006.01) *A61P 3/00* (2006.01)
*A61P 35/00* (2006.01)

(86) International application number:
**PCT/EP2006/068416**

(87) International publication number:
**WO 2007/054580 (18.05.2007 Gazette 2007/20)**

(54) **N-SULFAMOYL-N -BENZOPYRANPIPERIDINES AS INHBITORS OF CARBONIC ANHYDRASES**

N-SULFAMOYL-N BENZOPYRANPIPERIDINE ALS HEMMER VON KOHLENSTOFFANHYDRASEN

N-SULFAMOYL-N -BENZOPYRANPIPÉRIDINES EN TANT QU'INHIBITEURS D'ANHYDRIDASES CARBONIQUES

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **14.11.2005 EP 05110724**

(43) Date of publication of application:
**06.08.2008 Bulletin 2008/32**

(73) Proprietor: **Abbott Products GmbH**
**30173 Hannover (DE)**

(72) Inventors:
- **ANTEL, Jochen**
  **30173 Hannover (DE)**
- **WALDECK, Harald**
  **30173 Hannover (DE)**
- **SCHOEN, Uwe**
  **30173 Hannover (DE)**
- **GREGORY, Peter-Colin**
  **30173 Hannover (DE)**
- **WURL, Michael**
  **30173 Hannover (DE)**
- **FIRNGES, Michael**
  **30173 Hannover (DE)**
- **REICHE, Dania**
  **30173 Hannover (DE)**
- **REINECKER, Uwe**
  **30173 Hannover (DE)**
- **SANN, Holger**
  **30629 Hannover (DE)**

(74) Representative: **Gosmann, Martin et al**
**Abbott Products GmbH**
**IP Department (PH-ZP)**
**Hans-Böckler-Allee 20**
**30173 Hannover (DE)**

(56) References cited:
**WO-A-95/30642 WO-A-2004/092179**

- **DATABASE WPI Section Ch, Week 200537 Derwent Publications Ltd., London, GB; Class B02, AN 2005-359210 XP002369295 -& JP 2005 119987 A (AJINOMOTO KK) 12 May 2005 (2005-05-12)**
- **WERMUTH ET AL: "The Practise of Medicinal Chemistry" PRACTICE OF MEDICINAL CHEMISTRY, 1996, pages 203-237, XP002190259**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to novel N-sulfamoyl-N'-benzopyranpiperidines and their physiologically acceptable acid addition salts, to pharmaceutical compositions comprising them, processes for their preparation, and their use for the treatment and/or prophylaxis and/or prevention of epilepsy, bipolar disorders, migraine, neuropathic pain, obesity, type II diabetes, metabolic syndrome, alcohol dependence, and/or cancer, and its concomitant and/or secondary diseases or conditions.

Some 4-oxospiro[benzopyran-2,4'-piperdines] and their uses as Class III antiarrhythmic agents are described by Elliott et al. (J. Med. Chem. 1992, 35, 3973 to 3976). Similar compounds and their uses as selective alpha1a-adrenergic receptor antagonists are also described by Nerenberg et al. (Bioorganic & Medical Chemistry Letters 1999, 9, 291 to 294).

Yamato et. al. (J. Med. Chem. 1981, 24, 194 to 198) disclose synthesis and structure-activity relationship of spiro[isochromanpiperidine] analogues for inhibition of histamine release.

Fletcher et al. (J. Med. Chem. 2002, 45, 492 to 503) report on 4-(phenylsulfonyl)piperdines, their synthesis and use as bioavailable 5-HT2A receptor antagonists.

European patent application EP 0 431 943 teaches substituted spirocycles and their use as Class III antiarrhythmic agents, and positive inotropic or cardiotonic agents. EP 0 431 943 is also concerned with pharmaceutical formulations comprising one or more of the novel compounds as active ingredient, either alone or in combination with one or more of a Class I, Class II or Class IV antiarrhythmic agent.

A method of discovering compounds suitable for the treatment and/or prophylaxis of obesity by inhibiting lipogenesis via the inhibition of carbonic anhydrases in mammals and humans is known from document WO 02/07821.

WO 95/30642 describes a combinatorial dihydrobenzopyran library and the use of the compounds contained in the library as inhibitors of carbonic anhydrase isozymes for the treatment of ocular diseases, such as glaucoma. The compounds of WO 95/30642 do not contain any sulfamoyl-group.

JP 2005-119987 discloses acyl sulfonamide derivatives which are ACC inhibitors. The compounds disclosed are useful for the treatment of hyperlipemia induced by obesity and fatness.

WO 2004/092179 discloses spiro derivatives which are antioxidants. The compounds are also useful as therapeutic agents for kidney diseases or cerebrovascular disorders, as depressants for retinal oxidation disorders, and as lipoxygenase inhibitors.

It is an object of the present invention to provide novel medicaments for the treatment and/or prophylaxis and/or prevention of epilepsy, bipolar disorders, migraine, neuropathic pain, obesity, type II diabetes, metabolic syndrome, alcohol dependence, and/or cancer, and its concomitant and/or secondary diseases or conditions which are very effective and can be obtained in simple manner.

It has now surprisingly been found that certain novel N-sulfamoyl-N'-benzopyranpiperidines and their physiologically acceptable acid addition salts are suitable for the treatment and/or prophylaxis and/or prevention of epilepsy, bipolar disorders, migraine, neuropathic pain, obesity, type II diabetes, metabolic syndrome, alcohol dependence, and/or cancer, and its concomitant and/or secondary diseases or conditions.

The subject of the invention is a compound of general Formula I,

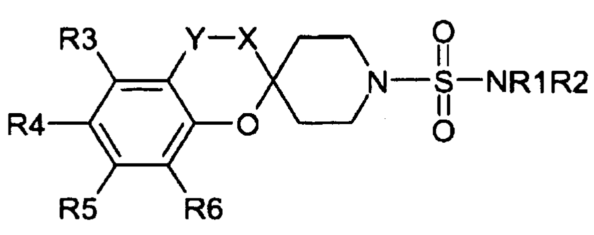

I

wherein

R1 and R2 are independently selected from the group consisting of: hydrogen, $C_1$ to $C_4$ alkyl, $C_4$ to $C_7$ cycloalkyl; or;

R1 and R2 together form a 5 or 6-membered ring which optionally may contain from 1 to 2 heteroatoms independently selected from the group consisting of nitrogen and/or oxygen atoms and which also may be substituted by optionally substituted aryl, optionally substituted heteroaryl or arylenehalogenalkyl;

R3 to R6 are independently selected from the group consisting of: hydrogen; halogen; $C_1$ to $C_4$ alkyl; $C_2$ to $C_4$ alkenyl, optionally substituted with aryl; $C_1$ to $C_4$ alkoxy; $C_1$ to $C_4$ alkoxy substituted with halogen provided that the alpha-carbon atom is substituted by no other halogen than fluorine if any; $C_2$ to $C_4$ alkinyl; $C_1$ to $C_4$ $NSO_2$alkyl; $NH_2$; $NO_2$; $C_1$ to $C_4$ aminoalkyl; $C_2$ to $C_8$ aminodialkyl; cyano; oxyaryl; oxyalkylenearyl; oxyarylenealkyl; oxyalkylenearylenealkoxy; $C_2$ to $C_8$ ester; $C_1$ to $C_8$ amido; $C_2$ to $C_8$ oxyalkylenecarbonylalkyl; $C_2$ to $C_8$ oxyalkyleneoxyalkyl; $C_1$ to $C_4$ amidooxyalkyl; aryl,

optionally substituted; heteroaryl, optionally substituted; condensed aryl, optionally substituted; condensed heterary1, optionally substituted; or;

R3 and R6 have the same meaning as above and wherein R4 and R5 together form a 5 or 6-membered ring which may optionally contain from 1 to 3 heteroatoms independently selected from the group consisting of: nitrogen, oxygen and sulfur and which may optionally bear 1 or 2 double bonds, and which also may contain a carbonyl group and which also may be substituted by $C_1$ to $C_4$ alkyl, $C_1$ to $C_4$ halogenalkyl, optionally substituted aryl, and/or optionally substituted heteroaryl; or;

R5 and R6 have the same meaning as above and wherein R3 and R4 together form a 5 or 6-membered ring which may optionally contain from 1 to 3 heteroatoms independently selected from the group consisting of: nitrogen, oxygen and sulfur and which may optionally bear 1 or 2 double bonds, and which also may contain a carbonyl group and which also may be substituted by $C_1$ to $C_4$ alkyl, $C_1$ to $C_4$ halogenalkyl, optionally substituted aryl, and/or optionally substituted heteroaryl; or;

R3 and R4 have the same meaning as above and wherein R5 and R6 together form a 5 or 6-membered ring which may optionally contain from 1 to 3 heteroatoms independently selected from the group consisting of: nitrogen, oxygen and sulfur and which may optionally bear 1 or 2 double bonds, and which also may contain a carbonyl group and which also may be substituted by $C_1$ to $C_4$ alkyl, $C_1$ to $C_4$ halogenalkyl, optionally substituted aryl, and/or optionally substituted heteroaryl;

Y-X is selected from the group consisting of: HC=CH, $CH_2$-$CH_2$, O=C-$CH_2$, and (HO)(H)C-$CH_2$;

and its physiologically acceptable acid addition salt.

Compounds of general Formula I can be used for the treatment and/or prophylaxis and/or prevention and/or inhibition of epilepsy, bipolar disorders, migraine, neuropathic pain, obesity, type II diabetes, metabolic syndrome, alcohol dependence, and/or cancer, and its concomitant and/or secondary diseases or conditions.

More specifically, in compounds of general Formula I R1 and R2 are independently selected from the group consisting of: hydrogen, $C_1$ to $C_4$ alkyl, $C_4$ to $C_7$ cycloalkyl, or, R1 and R2 together form a 5 or 6-membered ring which optionally may contain from 1 to 2 heteroatoms independently selected from the group consisting of nitrogen and/or oxygen atoms and which also may be substituted by optionally substituted aryl, optionally substituted heteroaryl, or arylenehalogenalkyl; R3 to R6 are independently selected from the group consisting of: hydrogen, flourine, chlorine, bromine, methyl, ethyl, propyl, butyl, ethylene, propylene, methoxy, ethoxy, propoxy, ethinyl, propinyl, butinyl, $NSO_2CH_3$, $NH_2$, $NO_2$, aminomethyl, aminoethyl, aminopropyl, aminobutyl, aminodimethyl, aminodiethyl, aminodipropyl, aminodibutyl, cyano, oxyphenyl, oxybenzyl, oxyethylenephenyl, oxyphenylenemethyl, oxyphenylenemethoxy, acetyl, amidomethyl, amidoethyl, oxymethylenecarbonylmethyl, oxyethylenecarbonylmethyl, oxymethylenecarbonylethyl, oxyethylenecarbonylethyl, oxymethyleneoxymethyl, oxymethyleneoxyethyl, oxyethyleneoxymethyl, oxyethyleneoxyethyl, amidooxymethyl, and amidooxyethyl; and Y-X is selected from the group consisting of: HC=CH, $CH_2$-$CH_2$, O=C-$CH_2$, and (HO)(H)C-$CH_2$.

Preferred compounds of general Formula I of the present invention are those R1 and R2 are both H; R3 to R6 are independently selected from the group consisting of: hydrogen, halogen and $C_1$ to $C_4$ alkoxy; and Y-X is O=C-$CH_2$.

Other preferred compounds of general Formula I of the present invention are those wherein R4 is selected from the group consisting of: hydrogen, chlorine, bromine, and methoxy; and R5 is selected from the group consisting of: hydrogen and bromine.

In another embodiment of the present invention, compounds of present formula I are preferred R1 and R2 are independently selected from the group consisting of: hydrogen, $C_1$ to $C_4$ alkyl, $C_4$ to $C_7$ cycloalkyl, or,

R1 and R2 together form a 5 or 6-membered ring which optionally may contain from 1 to 2 heteroatoms independently selected from the group consisting of nitrogen and/or oxygen atoms and which also may be substituted by optionally substituted aryl, optionally substituted heteroaryl or arylenehalogenalkyl;

R3, R5 and R6 are independently selected from the group consisting of: hydrogen; halogen; $C_1$ to $C_4$ alkyl; $C_2$ to $C_4$ alkenyl; C1 to $C_4$ alkoxy; $C_1$ to $C_4$ alkoxy substituted with halogen provided that the alpha-carbon atom is substituted by no other halogen than fluorine if any; $C_2$ to $C_4$ alkinyl; $C_1$ to $C_4$ $NSO_2$alkyl; $NH_2$; $NO_2$; $C_1$ to $C_4$ aminoalkyl; $C_2$ to $C_8$ aminodialkyl; cyano; oxyaryl; oxyalkylenearyl; oxyarylenealkyl; oxyalkylenearylenealkoxy; $C_2$ to $C_8$ ester; $C_1$ to $C_8$ amido; $C_2$ to $C_8$ oxyalkylenecarbonylalkyl; $C_2$ to $C_8$ oxyalkyleneoxyalkyl; and; $C_1$ to $C_4$ amidooxyalkyl;

R4 is selected from the group consisting of:

(a) phenyl, optionally substituted with one to three substituents independently selected from the group consisting of: hydrogen, amino, halogen, $C_1$ to $C_4$ alkyl, $C_1$ to $C_4$ alkoxy, oxyaryl, $C_1$ to $C_4$ mercapto, C(O)H, trifluormethyl, -N-C(O)$C_{1-4}$alkyl, $C_2$ to $C_4$ alkenyl, C(O)$C_{1-4}$alkyl, and aryl;

(b) 1-naphthyl, 2-naphthyl, 2-pyridyl, 3-pyridyl, 4-pyridyl, 2-thiophenyl, 3-thiophenyl, quinoline, isoquinoline, benzo [b]thiophene, 1,3-dihydro-benzo[c]thiophene, 1-dibenzofuran, 2-dibenzofuran, 3-dibenzofuran, and, 4-dibenzofuran, each optionally substituted with one to three substituents selected from the group consisting of: hydrogen,

amino, halogen, $C_1$ to $C_4$ alkyl, $C_1$ to $C_4$ alkoxy, oxyaryl, $C_1$ to $C_4$ mercapto, C(O)H, trifluormethyl, -N-C(O)$C_{1-4}$alkyl, $C_2$ to $C_4$ alkenyl, C(O)$C_{1-4}$alkyl, and aryl; and;

Y-X is selected from the group consisting of: HC=CH, $CH_2$-$CH_2$, O=C-$CH_2$, and (HO)(H)C-$CH_2$.

More preferred are compounds wherein R1, R2, R3, R5 and R6 are each hydrogen; and R4 is selected from the group consisting of:

(a) phenyl, optionally substituted with one to three substituents independently selected from the group consisting of: hydrogen, amino, halogen, $C_1$ to $C_4$ alkyl, $C_1$ to $C_4$ alkoxy, oxyaryl, C1 to C4 mercapto, C(O)H, trifluormethyl, -N-C(O)$_{1-4}$alkyl, C2 to C4 alkenyl, C(O)$_{1-4}$alkyl, aryl;

(b) 1-naphthyl, 2-naphthyl, 2-pyridyl, 3-pyridyl, 4-pyridyl, 2-thiophenyl, 3-thiophenyl, quinoline, isoquinoline, benzo [b]thiophene, 1,3-dihydrobenzo[c]thiophene, 1-dibenzofuran, 2-dibenzofuran, 3-dibenzofuran, 4-dibenzofuran; and;

Y-X is O=C-$CH_2$.

Where in the compounds of the present invention or in other compounds described within the context of the present invention substituents are or contain $C_1$ to $C_4$-alkyl, $C_2$ to $C_4$-alkenyl, $C_1$ to $C_4$-alkoxy, $C_1$ to $C_4$-alkoxy substituted with halogen provided that the alpha-carbon atom is substituted by no other halogen than fluorine if any, $C_2$ to $C_4$-alkinyl, aminoalkyl, $C_2$ to $C_8$ dialkyl, oxyaryl, oxyalkylenearyl, oxyarylenealkyl, oxyalkylenearylenealkoxy, $C_2$ to $C_8$ ester, $C_1$ to $C_8$ amido, $C_2$ to $C_8$ oxyalkylenecarbonylalkyl, $C_2$ to $C_8$ oxyalkyleneoxyalkyl, or $C_1$ to $C_4$ amidooxyalkyl, these may each be straight-chain or branched. Where substituents in compounds of general Formula I stand for halogen, fluorine, chlorine, bromine or iodine are suitable. Chlorine and bromine are preferred.

Aryl and/or arylene preferably stands for phenyl and phenylene. Where aryl and/or arylene is optionally substituted, phenyl and/or phenylene is substituted with one to three substituents independently selected from the group consisting of: hydrogen, amino, halogen, $C_1$ to $C_4$ alkyl, $C_1$ to $C_4$ alkoxy, oxyaryl, $C_1$ to $C_4$ mercapto, C(O)H, trifluormethyl, -N-C(O)$C_{1-4}$alkyl, $C_2$ to $C_4$ alkenyl, C(O)$C_{1-4}$alkyl, aryl, cyano, nitro and $C_1$ to $C_4$ alkylsulfonyl are preferred substituents. More preferred are halogen, $C_1$ to $C_4$ alkyl, $C_1$ to $C_4$ alkoxy and trifluoromethyl. Unsubstituted phenyl is also an alternative.

Heteraryl preferably stands for pyridyl and pyridylene, in particular 2-pyridyl, 3-pyridyl or 4-pyridyl; pyrimidinyl and pyrimidinylene, in particular 2-pyrimidinyl, 5-pyrimidinyl, 2-pyrimidinylene and 5-pyrimidinylene; oxazolyl; thiazolyl; thiophenyl, in particular 2-thiphenyl, 3-thiophenyl; and furanyl, in particular, 2-furanyl and 3-furanyl. Phenyl, pyridyl and pyrimidinyl are more preferred. Where heteroaryl is optionally substituted, it is substistuted with halogen, trifluoromethyl, cyano, $C_1$ to $C_4$ alkyl and $C_1$ to $C_4$ alkoxy.

Condensed aryl and condensed heteroaryl preferably stands for naphthyl, in particular 1-naphthyl, 2-naphthyl; quinolinyl; isoquinolinyl; 1,2,3,4-tetrahydroisoquinolinyl; indoly; isoindolinyl; benzo[b]thiophene, 1,3-dihydro-benzo[c] thiophene, 1-dibenzofuran, 2-dibenzofuran, 3-dibenzofuran, 4-dibenzofuran. Where condensed heteroaryl is optionally substituted, it is substistuted with halogen, trifluoromethyl, cyano, $C_1$ to $C_4$ alkyl and $C_1$ to $C_4$ alkoxy.

Physiologically compatible acid addition salts of compounds of general Formula I are their conventional salts with inorganic acids, for example sulphuric acid, phosphoric acids or hydrohalic acids, preferably hydrochloric acid, or with organic acids, for example lower aliphatic monocarboxylic, dicarboxylic or tricarboxylic acids such as maleic acid, fumaric acid, lactic acid, tartaric acid, citric acid, or with sulphonic acids, for example lower alkanesulphonic acids such as methanesulphonic acid or trifluoromethanesulphonic acid, or benzenesulphonic acids optionally substituted in the benzene ring by halogen or lower alkyl, such as p-toluenesulphonic acid. Hydrochloric acid salts of the compounds of general Formula I are preferred.

In general, compound of general Formula I can be prepared by reacting a benzopyrane compound of general Formula II,

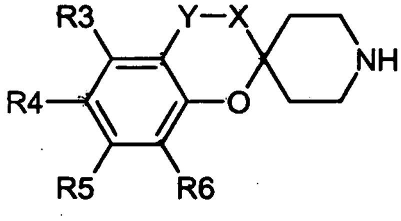

II

wherein R3 to R6, X and Y have the above meanings, with sulfamide, or, with a sulfamoylchloride, which is optionally protected with a protecting group, preferably tert.-butyloxycarbonyl, of general Formula IV,

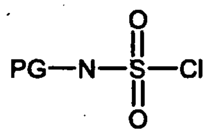

IV

and if desired converting resulting free bases of general Formula I into their physiologically acceptable salts, or converting salts of the compounds of general formula I into the free bases of general Formula I.

Compounds of general Formula I can also be produced by reacting a benzopyrane of general Formula II

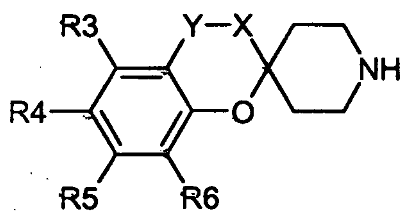

II

wherein R3 to R6, X and Y have the above meanings, with a compound of general Formula III

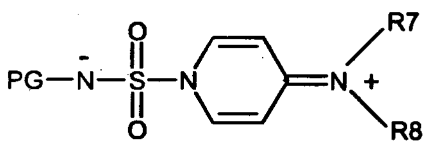

III

wherein R7 and R8 $C_1$ to $C_6$ alkyl and/or $C_3$ to $C_8$ cycloalkyl, wherein PG denotes a protecting group, preferably tert.-butyloxycarbonyl, and subsequently cleaving off the PG-group under suitable, preferably acidic, conditions from the obtained intermediate compound, and if desired converting resulting free bases of general Formula I into their physiologically acceptable acid addition salts, or converting the acid addition salts of the compounds of general Formula I into the free bases of general Formula I. In a preferred embodiment of this process, R7 and R8 are both methyl.

Compounds of general Formula I wherein R1 and R2 are not hydrogen can be produced by reacting a benzopyrane of general Formula II

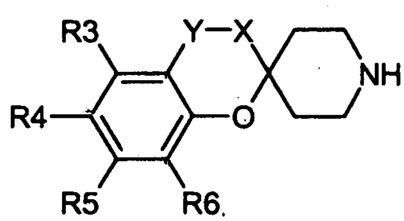

II

wherein R3 to R6, X and Y have the above meanings, with a sulfamoylchloride, which is preferably protected with a protecting group, preferably tert.-butyloxycarbonyl, of general Formula IV,

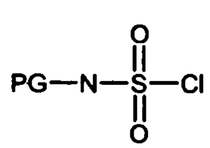

IV

and subsequently cleaving off the protecting group under suitable, preferably acidic, conditions from the obtained intermediate product, and if desired converting resulting free bases of general Formula I into their physiologically acceptable acid addition salts, or converting the acid addition salts of the compounds of general Formula I into the free bases of general Formula I.

Compounds of general Formula I wherein R1 and R2 are not hydrogen can be produced by reacting a benzopyrane of general Formula II

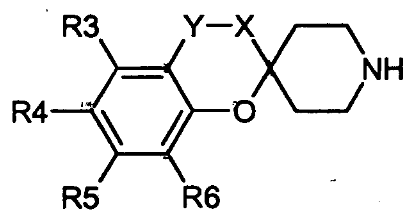

II

wherein R3 to R6, X and Y have the above meanings, with a sulfamoylchloride of general Formula IVa,

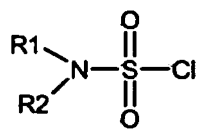

IVa

wherein R1 and R2 have the above meanings, and if desired converting resulting free bases of Formula I into their physiologically acceptable acid addition salts, or converting the acid addition salts of the compounds of Formula I into the free bases of Formula I.

Compounds of general Formula I wherein at least one of R3 to R6 is aryl, optionally substituted; heteroaryl, optionally substituted; condensed aryl, optionally substituted; condensed heterary1, optionally substituted, can be prepared by reacting a compound of Formula I wherein at least one of R3 to R6 is bromo, chloro, or, iodo, preferably bromo or chloro, more preferably bromo, with a compound of formula IX

$W - B(OH)_2$ IX

wherein W is selected from the group consisting of: aryl, optionally substituted; heteroaryl, optionally substituted; condensed aryl, optionally substituted; condensed heterary1, optionally substituted. This reaction requires the presence of Palladium(0) (generated for instance from Palladium-(II)-acetat) and a phosphine ligand, e.g. a biphenyl phosphan catalyst as disclosed in Buchwald et al., Angew. Chem. Int. Ed., 2004, 43, 1871-1876. Suitable catalysts are for instance any of:

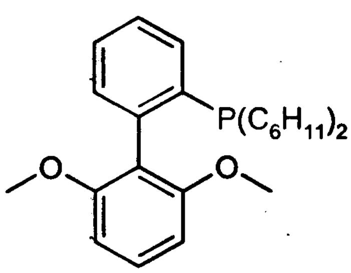

"S-Phos"

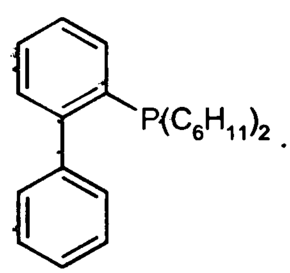

"DCPB"

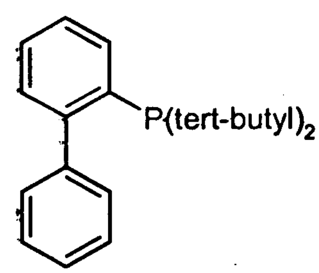

"JohnPhos"

More specifically, compounds of general Formula I can be produced in the following manner:

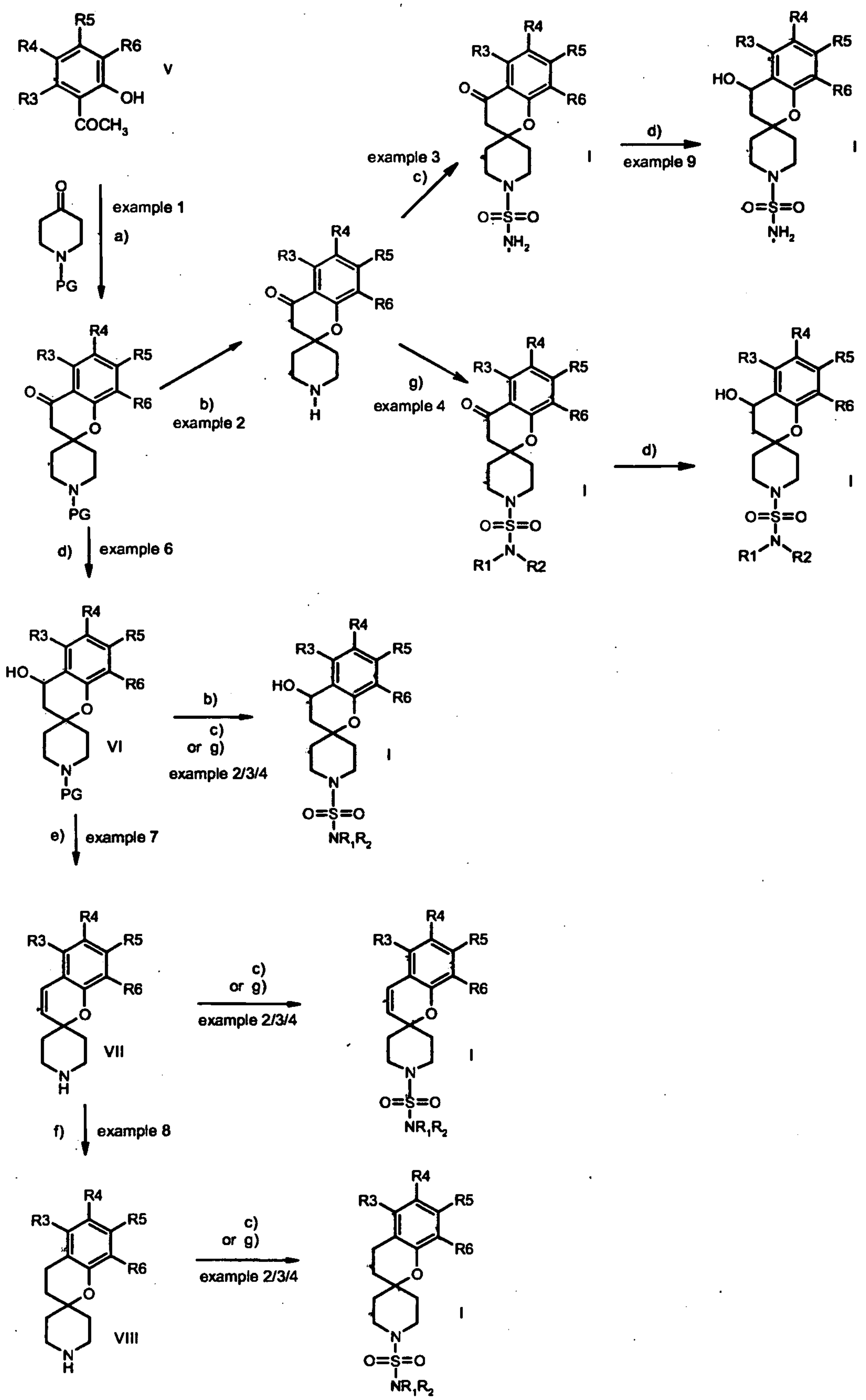
V
example 1
a)
b)
example 2
example 3
c)
I
d)
example 9
I
g)
example 4
I
d)
I
d)
example 6
VI
b)
c)
or g)
example 2/3/4
I
e)
example 7
VII
c)
or g)
example 2/3/4
I
f)
example 8
VIII
c)
or g)
example 2/3/4
I

and if desired converting resulting free bases of general Formula I into their physiologically acceptable salts, or converting salts of the compounds of general Formula I into the free bases of general Formula I.

Any compound of Formula I, obtained by any process as described in the scheme above, can be further reacted to the give a compound of Formula I wherein any of R3 to R6 is represented by aryl, optionally substituted; heteroaryl, optionally substituted; condensed aryl, optionally substituted; condensed heteraryl, optionally substituted..

In process step a), the reaction can be carried in an organic solvent which is inert under the reaction conditions, in particular in a solvent such as methanol. Suitable reaction temperatures are between room temperature and the boiling point of the solvent, preferably between 30˚C and 65˚C. The crude product can optionally be purified on a suitable gel, e.g., silica gel, if needed to obtain the spiro compound in good yield.

In process step b), the protecting group, preferably tert.-butyloxycarbonyl, can subsequently be cleaved off in a known manner in acidic media, e.g. in an ethanolic solution of hydrochloric acid or even with concentrated hypochlorid acid. The yield can be optimized by extracting the reaction crude with a dipolar-aprotic solvent such as chloroform, dichloromethane or in a mixture of such solvents.

In process step c), the reaction can be carried out in an organic solvent which is inert under the reaction conditions, in particular in an aprotic solvent such as toluene or xylene or in a mixture of such solvents. Suitable reaction temperatures are between room temperature and the boiling point of the solvent or solvent mixture, preferably between 60˚C and 100˚C. After cooling to room temperature, the solvents were evaporated and the remaining solids dissolved in an organic inorganic solvent mixture such as methanol, tetrahydrofuran and water. Removal of the solvents leads to compounds of general Formula I in good yield.

In process step d), the reaction can be carried out in an organic solvent which is inert under the reaction conditions, in particular in a protic solvent such as methanol or ethanol or in a mixture of such solvents. Suitable reaction temperatures are between 0˚C and room temperature, preferably between 10˚C and 20˚C. The solvents were evaporated and the remaining solids dissolved in an organic inorganic solvent mixture such as ethyl acetate and water. Removal of the solvents leads to compounds of general Formula VI in good yield.

In process step e), the reaction can be carried out in an organic solvent which is inert under the reaction conditions, in particular in an aprotic solvent such as toluene or xylene or in a mixture of such solvents. Suitable reaction temperatures are between room temperature and the boiling point of the solvent or solvent mixture, preferably between 60˚C and 100˚C. After cooling to room temperature, the solvents were evaporated and the remaining solids dissolved in an organic inorganic solvent mixture such as ethyl acetate and water. Removal of the solvents leads to compounds of general Formula VII in good yield.

In process step f), the reaction can be carried out in an organic solvent which is inert under the reaction conditions, in particular in an protic solvent such as methanol or ethanol or in a mixture of such solvents. Suitable reaction temperatures are between room temperature and the boiling point of the solvent or solvent mixture, preferably between 20˚C and 40˚C. After cooling to room temperature, the solvents were evaporated and the remaining solids dissolved in an organic inorganic solvent mixture such as ethyl acetate and water. Removal of the solvents leads to compounds of general Formula VIII in good yield.

In process step g) the benzopyran derivative is reacted with the respective sulfamoylchloride in an inert organic solvent, e.g., dichloromethane or THF, preferably in the presence of a base, e.g., triethylamine. In a preferred embodiment, the benzopyrane is used in excess. To remove excess or reagents, polymeric isocyanate and trisamine can be added.

Starting materials, such as compounds of general Formula V,

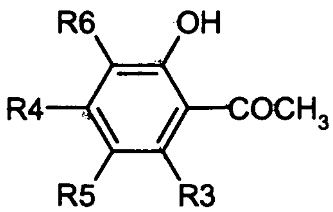

V

for the above reaction pathways are either commercially available, or can be prepared by one of the reaction pathways described in the following examples.

1. Para-anisidine was acylated at the amino center with acetic anhydride in dichloromethane and the product was obtained in 91% yield. Then, Friedel Craft's acylation was carried out to get the hydroxyl acetophenone with acetyl chloride in the presence of anhydrous aluminium chloride in dichloromethane to give the product in 70% yield. Nitration of acetanilide derivative was carried out with nitric acid in aqueous acetic acid to get the product in 45%

yield. Acetyl group of acetamido functionality was removed by refluxing in dilute hydrochloric acid for 2.5 h to get the aniline derivative in quantitative yield. Deamination was done by diazotization and treating the diazonium salt with ethanol to get the 3-nitro-2-hydroxyacetophenone. The nitro group was reduced by heating the reaction mixture in ethyl acetate with tin in hydrochloric acid resulting in the formation of corresponding amino compound. Lastly, Sandemeyer reaction was carried out to get the desired bromo derivative Va in 25% yield.

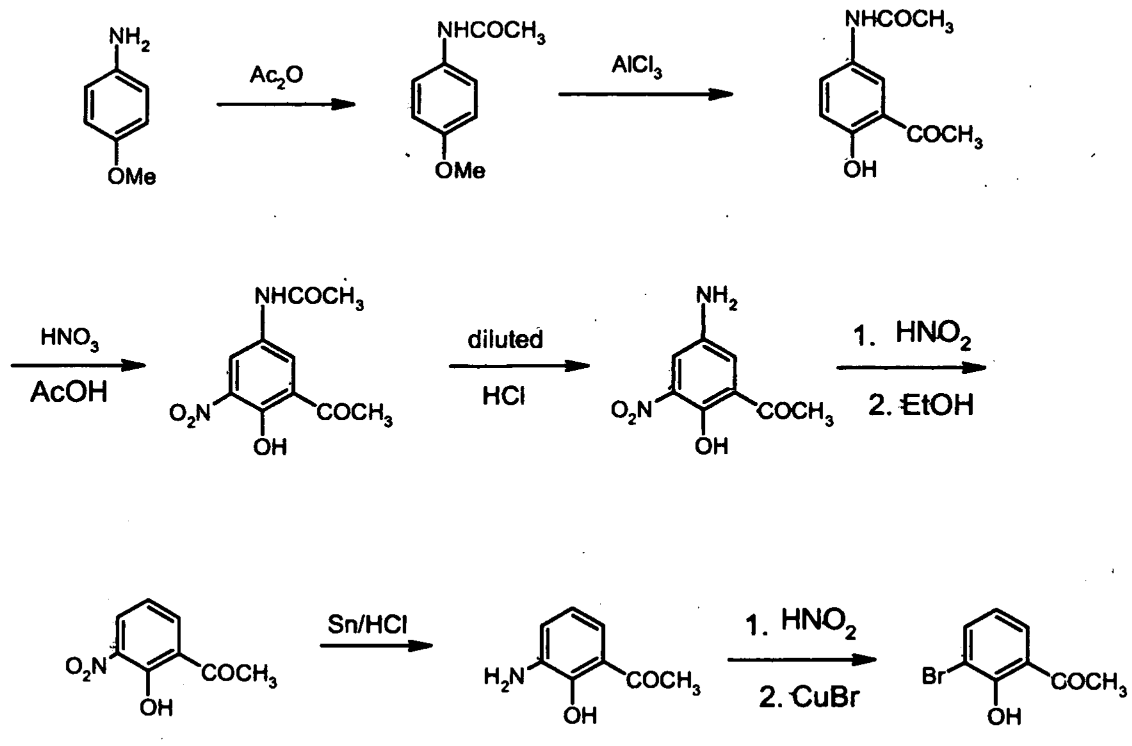


Va

2. 2-hydroxy-4-bromoacetophenone was synthesized through the schematic pathway shown below. 3-Bromophenol was acylated with acetic anhydride in the presence of catalytic quantity of sulfuric acid to get the acylated product in quantitative yield. Fries migration of the same was carried out by heating with anhydrous aluminium chloride in o-dichlorobenzene for 8 h to get the product in 70 % yield, which is condensed with N-bocpiperidone in methanol in the presence of pyrrolidine and desired product Vb was obtained in 65 % yield.

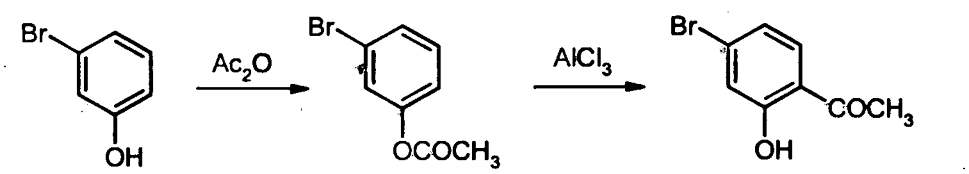


Vb

3. 2-hydroxy-5-cyanoacetophenone was obtained by acylation of 3-cyanophenol and carrying out Fries Migration. Acylation was achieved by treating the cyanophenol with acetic anhydride in the presence of catalytic amount of concentrated sulphuric acid leading to 59% of the pure acetylated product. Further, the phenyl acetate was subjected to Fries Migration conditions, i.e, by heating at 180-185 ˚C with aluminium chloride for 3 h and the desired product Vc was isolated in 45 % yield.

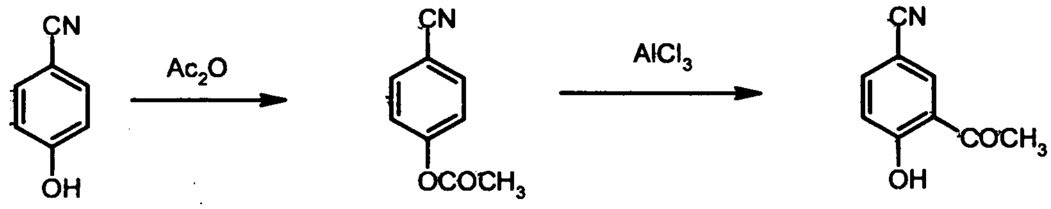


Vc

4. 4-amino-2-hydroxyacetophenone was obtained through the pathway given below. 3-Aminophenol is used as the raw material and condensed with acetic anhydride in the presence of pyridine to get the diacetyl derivative in 80% yield. Fries migration was carried out with this compound by heating with anhydrous aluminium chloride at 170-180 ˚C for 8 h to get the acetophenone derivative which was hydrolyzed without isolation by refluxing in 2N HCl to get the product Vd in 60% yield

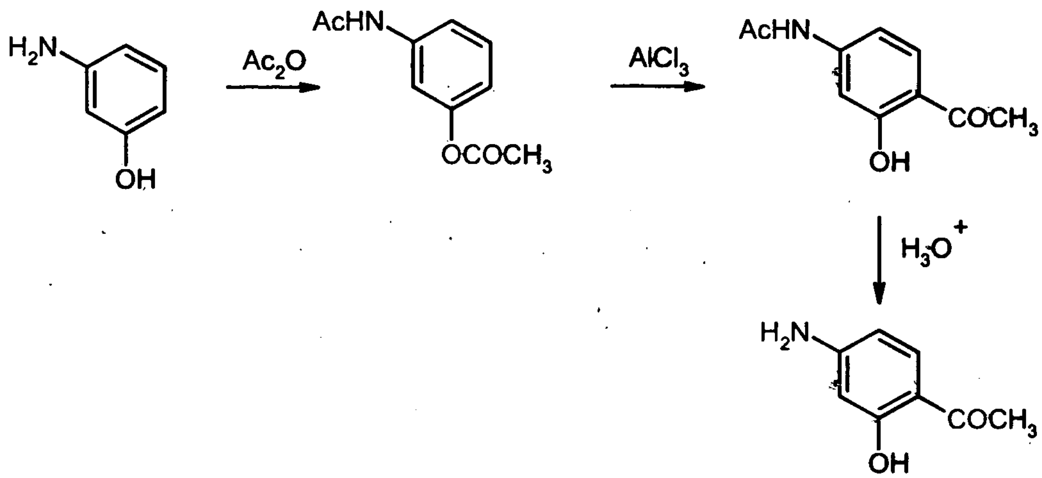


Vd

5. p-Anisidine is acylated at the amino center with acetic anhydride in dichloromethane and the acylated product was obtained in 91% yield . Then, Friedel Craft's acylation was carried out to get the hydroxyacetophenone derivative with acetyl chloride in the presence of anhydrous aluminium chloride in DCM. The intermediate was isolated in 70% yield. 5-Acetamido-2-hydroxyacetophenone was hydrolyzed in 2N hydrochloric solution by refluxing for 6 h, yielding 94% of 5-amino-2-hydroxyacetophenone.

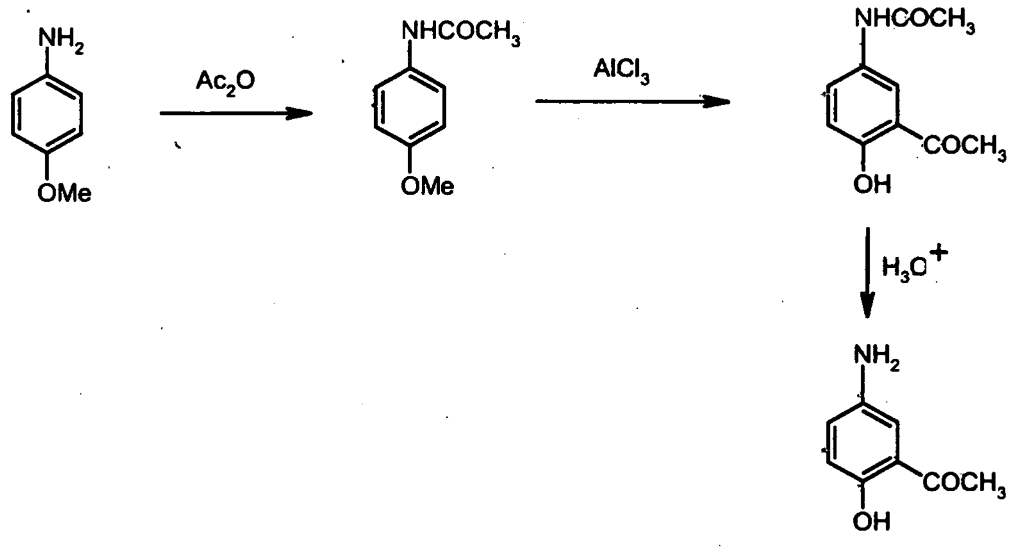


Ve

Other compounds of general Formula V can be prepared in a similar manner by replacing the starting materials respectively.

In yet another aspect, the present invention also relates to a compound of formula I for use in a method of treatment and/or prophylaxis and/or prevention of epilepsy, bipolar disorders, migraine, neuropathic pain, obesity, type II diabetes, metabolic syndrome, alcohol dependence, and/or cancer, and its concomitant and/or secondary diseases or conditions in mammals and humans, comprising administering to a subject in need thereof a therapeutically effective amount of a compound of general Formula I or its physiologically compatible acid addition salts.

Obesity according to the present invention is meant to comprise any increase in body fat that results in increased bodyweight, comprising as a preferred alternative but not limited to the medical definition of obesity. The invention thus also relates to non-medical weight loss, such as cosmetic weight loss and includes improving bodily appearance in general. Further, the term obesity also is meant to comprise drug induced obesity and/or juvenile obesity.

The concomitant diseases of obesity and its concomitant and/or secondary diseases or conditions in mammals and humans according to the invention include in particular the metabolic syndrome and/or syndrome X and cardiovascular diseases.

The term "metabolic syndrome" as used in this application is meant to cover a complex of clinical pictures which - besides central obesity - mainly comprises hypertension, in particular arterial hypertension; insulin resistance, in particular diabetes mellitus type II; glucose intolerance; dyslipoproteinaemia, in particular as hypertriglyceridaemia, accompanied by dyslipoproteinaemia occurring with lowered HDL-cholesterol, and also hyperuricaemia, which can lead to gout. According to information from the American Heart Association, the metabolic syndrome is closely linked to insulin resistance. Some people are genetically predisposed to insulin resistance. Acquired factors, such as excess body fat and physical inactivity, can elicit insulin resistance and the metabolic syndrome in these people. Most people with insulin resistance have central obesity. The biologic mechanisms at the molecular level between insulin resistance and metabolic risk factors are not fully understood and appear to be complex. One group of people at risk for developing metabolic syndrome are those with diabetes who have a defect in insulin action and cannot maintain a proper level of glucose in their blood. Another is people, mainly those with high blood pressure, who are nondiabetic and insulin-resistant but who compensate by secreting large amounts of insulin. This condition is known as hyperinsulinemia. A third group is heart attack survivors who, unlike hypertensives, have hyperinsulinemia without having abnormal glucose levels. The metabolic syndrome has become increasingly common in higher developed countries like the United States, where it is estimated that about 20-25 percent of US adults have it. There are no well-accepted criteria for diagnosing the metabolic syndrome.

The criteria proposed by the Third Report of the National Cholesterol Education Program (NCEP) Expert Panel on Detection, Evaluation, and Treatment of High Blood Cholesterol in Adults (Adult Treatment Panel III) are the most current and widely used. According to the ATP III criteria, the metabolic syndrome is identified by the presence of three or more of these components:

- Central obesity as measured by waist circumference (Men - Greater than 40 inches; Women - Greater than 35 inches).

- Fasting blood triglycerides greater than or equal to 150 mg/dL.
- Blood HDL cholesterol (Men - Less than 40 mg/dL; Women - Less than 50 mg/dL)
- Blood pressure greater than or equal to 130/85 mmHg.
- Fasting glucose greater than or equal to 110 mg/dL.

The term "syndrome X" is closely related to the term "metabolic syndrome" and usually is supposed to denominate the identical disease or condition. According to information from the American Heart Association, the term "Syndrome X" refers, however, additionally to a heart condition where chest pain and electrocardiographic changes that suggest ischemic heart disease are present, but where there are no angiographic findings of coronary disease. Patients with cardiac syndrome X also sometimes have lipid abnormalities.

The term "cardiovascular diseases" in conjunction with obesity is usually understood to mean coronary heart disease, which can lead to heart failure, cerebrovascular diseases, which may for example be accompanied by an increased risk of strokes, and peripheral occlusive arterial disease.

Due to their inherent properties, the compounds of general Formula I or their physiologically compatible acid addition salts are also expected to be useful in the treatment of diabetic conditions or diseases which are unrelated to obesity. Such diabetic conditions or diseases comprise e.g. diabetes mellitus type II, diabetic neuropathy, diabetic retinopathy, diabetic nephropathy, diabetic microangiopathy or diabetic macroangiopathy.

Further concomitant and/or secondary diseases of obesity may be gall-bladder diseases such as formation of gallstones, sleep apnoea syndrome, orthopaedic complications such as osteoarthritis and psychosocial disorders.

The compounds of general Formula I are further deemed to be useful as anticonvulsants for the prophylaxis or treatment of epilepsy in mammals and humans.

The compounds of general Formula I according to the invention are inhibitors of mammalian carbonic anhydrases, in particular of human carbonic anhydrase isozymes of subtypes II and/or V (= hCA II and/ or hCA V).

*Pharmacological Test Methods*

The example numbers quoted in the pharmacological test methods relate to the preparation examples described below.

1. In vitro inhibition of human carbonic anhydrase isoenzyme II (hCA II)

The test compounds of general Formula I in 96 well microplates were diluted with aqua bidest by using an automatic pipettor (CyBiWell®). From the different dilution plates, aliquots of 20 μl were transferred to the 96 well black assay plates with a pipetting station (Tecan Genesis®). In a second step, 148 μl of potassium phosphate buffer (20 mM, pH 7.4) was added, and as a third step, 20 μl of enzyme solution (1 μM human carbonic anhydrase isoenzyme II from erythrocytes (Sigma-Aldrich), dissolved in potassium phosphate buffer) incubated for 60 min at room temperature and the fluorescence signal (Tecan Ultra® fluorescence reader; excitation wavelength: 280 nm; emission wavelength: 465 nm) read at the end of the preincubation period (FLU-1). After the preincubation time, 20 μl of aqueous dansylamide solution (1 mM dansylamide (Sigma-Aldrich), dissolved in hydrochloric acid) were added and the fluorescence signal read every 10 min for a period of 60 min at 37°C. For calculation, the fluorescence data of the time point 60 min (FLU-2) were used. The total volume of assay mixture amounted to 208 μl. The final concentration of carbonic anhydrase II was $10^{-7}$ M/L, of dansylamide $2.25 \times 10^{-6}$ and of compounds from $10^{-8}$ M/L up to $10^{-5}$ M/L. Final concentration of DMSO as compound solvent was 0.1 mM. Each microplate also contained blanks without compound and enzyme, controls without compound and ethoxzolamide (final concentration $5 \times 10^{-8}$ M/L). All data reflect single measurements. Data were expressed as % inhibition after calculation by the formula:

$$\%\ \text{inhibition} = 100((1-(FLU\text{-}2_{cpd}-FLU\text{-}2_{blank}-FLU\text{-}1_{cpd}+FLU\text{-}1_{blank})/(FLU\text{-}2_{control}-FLU\text{-}2_{blank}-FLU\text{-}1_{control}-FLU\text{-}1_{blank}))$$

The %inhibition data for each compound and the respective final concentrations were used for $IC_{50}$ calculations by using the Prism 4 software. Concentration action figures were calculated by applying the Prism algorithm for nonlinear regression (curve-fit): sigmoidal dose response with variable slope and the constraints: top: 100 and bottom 0.

In this test model, the test substances of general formula I listed in Table 1 below showed the $IC_{50}$ values given below:

Table 1: hCA II inhibiting effect of the test substances in vitro

| Example No. | $IC_{50}$ [μM] |
|---|---|
| 1 | 6,6 |
| 2 | 6,6 |
| 3 | 7,7 |
| 4 | 6,4 |

2. Acute in vivo food intake test in mice

The studies were carried out in male or female C57Bl/6 mice (n=8-12 per group). The mice were kept on an inverted 12/12h light/dark cycle (lights on 22:00). They were allowed food (high caloric diet) and water *ad libitum.* Food intake and water consumption was measured daily. The test compound of general Formula I was suspended in 1% methylcellulose in water and 2% (v/v) of Poloxamer 188 (Lutrol F68®) and administered by oral gavage at a dose of 100 mg/kg/day. One half of the dose was administered at 7.00-9.00 h; the remaining half of the dose was administered between 15.00-15.30 h.

In the test model described above, the test substances caused a decrease of the animals' 24h food intake to the percentages of food intake when compared to control as given in Table 2 below.

Table 2: Influence of test substances on food intake

| Example No. | food intake [% of control] |
|---|---|
| 1 | 83 |

The present invention further provides a pharmaceutical composition or medicament comprising a pharmacologically effective quantity of a compound of general Formula I or its physiologically compatible acid addition salts and further comprising conventional pharmaceutically acceptable auxiliaries and/or carriers.

Suitable pharmaceutically acceptable auxiliaries and/or carriers are well known in the art and include pharmaceutical grade starch, mannitol, lactose, magnesium stearate, sodium saccharin, talcum, cellulose, glucose, sucrose (or other sugar), magnesium carbonate, gelatin, oil, alcohol, detergents, emulsifiers or water (preferably sterile). The composition may be a mixed preparation of a composition or may be a combined preparation for simultaneous, separate or sequential use (including administration). The compounds according to the invention or their physiologically compatible acid addition salts for use in the aforementioned indications may be administered by any convenient method, for example by oral (including by inhalation), parenteral, mucosal (e.g. buccal, sublingual, nasal), rectal or transdermal administration and the compositions adapted accordingly. For oral administration, the compounds can be formulated as liquids or solids, for example solutions, syrups, suspensions or emulsions, tablets, capsules and lozenges. A liquid formulation will generally consist of a suspension or solution of the compound or physiologically acceptable salt in a suitable aqueous or non-aqueous liquid carrier(s) for example water, ethanol, glycerine, polyethylene glycol or an oil.

The formulation may also contain a suspending agent, preservative, flavouring or colouring agent. A composition in the form of a tablet can be prepared using any suitable pharmaceutical carrier(s) routinely used for preparing solid formulations. Examples of such carriers include magnesium stearate, starch, lactose, sucrose and microcrystalline cellulose. A composition in the form of a capsule can be prepared using routine encapsulation procedures. For example, powders, granules or pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule. Compositions for oral administration may be designed to protect the active ingredient against degradation as it passes through the alimentary tract, for example by an outer coating of the formulation on a tablet or capsule. Typical parenteral compositions consist of a solution or suspension of the compound or physiologically compatible acid addition salts in a sterile aqueous or non-aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration. Compositions for nasal or oral administration may conveniently be formulated as aerosols, drops, gels and powders.

Aerosol formulations typically comprise a solution or fine suspension of the active substance in a physiologically acceptable aqueous or non-aqueous solvent and are usually presented in single or multidose quantities in sterile form in a sealed container, which can take the form of a cartridge or refill for use with an atomising device. Alternatively the sealed container may be a unitary dispensing device such as a single dose nasal inhaler or an aerosol dispenser fitted with a metering valve which is intended for disposal once the contents of the container have been exhausted. Where the dosage form comprises an aerosol dispenser, it will contain a pharmaceutically acceptable propellant. The aerosol dosage forms can also take the form of a pump-atomiser. Compositions suitable for buccal or sublingual administration include tablets, lozenges and pastilles, wherein the active ingredient is formulated with a carrier such as sugar and acacia, tragacanth, or gelatin and glycerin. Compositions for rectal or vaginal administration are conveniently in the form of suppositories (containing a conventional suppository base such as cocoa butter), pessaries, vaginal tabs, foams or enemas. Compositions suitable for transdermal administration include ointments, gels, patches and injections including powder injections. Conveniently the composition is in unit dose form such as a tablet, capsule or ampoule. The pharmaceutical compositions according to the invention are useful in the treatment and/or prophylaxis and/or prevention of epilepsy, bipolar disorders, migraine, neuropathic pain, obesity, type II diabetes, metabolic syndrome, alcohol dependence, and/or cancer, and its concomitant and/or secondary diseases or conditions; other medical weight loss and non-medical related weight loss; and/or diabetic conditions or diseases.

The compounds of the present invention and their physiologically compatible acid addition salts are generally administered as pharmaceutical compositions which are important and novel embodiments of the invention because of the presence of the compounds disclosed herein. In embodiments of the invention, a pharmaceutical pack or kit is provided comprising one or more container(s) filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

Yet a further aspect of the invention provides a process for the manufacture of a pharmaceutical composition as described hereabove. The manufacture can be carried out by standard techniques well known in the art and involves combining a compound according to the invention and the pharmaceutically acceptable auxiliaries and/or carriers. The composition may be in any form including a tablet, a liquid, a capsule, and a powder or in the form of a food product, e.g. a functional food. In the latter case the food product itself may act as the pharmaceutically acceptable carrier.

The compound or composition is preferably administered to a patient in need thereof and in a quantity sufficient to prevent and/or treat the symptoms of the condition, disorder or disease. For all aspects of the invention, particularly medical ones, the administration of a compound or composition has a dosage regime which will ultimately be determined by the attending physician and will take into consideration such factors such as the compound being used, animal type, age, weight, severity of symptoms, method of administration, adverse reactions and/or other contraindications. Specific defined dosage ranges can be determined by standard design clinical trials with patient progress and recovery being fully monitored. Such trials may use an escalating dose design using a low percentage of the maximum tolerated dose in animals as the starting dose in man. The physiologically acceptable compounds of the invention will normally be administered in a daily dosage regimen (for an adult patient) of, for example, an oral dose of between 1 mg and 2000 mg, preferably between 30 mg and 1000 mg, e.g. between 10 and 250 mg or an intravenous, subcutaneous, or intramuscular dose of between 0.1 mg and 100 mg, preferably between 0.1 mg and 50 mg, e.g. between 1 and 25 mg of the compound of the general Formula I or a physiologically acceptable salt thereof calculated as the free base, the compound being administered 1 to 4 times per day. The compound used according to the invention can also be administered to children or juveniles while the individual dosage regimens in these cases will need to be particularly thoroughly adjusted by the physician and will usually comprise lower doses than will be administered to adults.

Suitably the compounds will be administered for a period of continuous therapy, for example for at least a week, but usually for a longer period of several weeks to several months. The invention also provides a cosmetic method (non-therapeutic) for maintaining a given weight, or for cosmetic weight loss, the method comprising the administration of a compound according to the other aspects of the invention, preferably in combination with a pharmaceutically acceptable carrier or diluent.

The compound or composition is preferably administered to a subject in need or in desideratum thereof and in a quantity sufficient to maintain a given weight or for cosmetic weight loss.

In still a further aspect, the compounds of general Formula I and their physiologically compatible acid addition salts may favourably be administered in combination with one or more active agents (as a pharmaceutical combination composition) selected from antidiabetics; antiobesity or appetite-regulating agents; cardiovascular active agents, in particular antihypertensives; diuretics; active agents altering lipid levels, in particular lipid-lowering agents; and active ingredients for the treatment and/or prevention of complications caused by diabetes or associated with diabetes.

Suitable antidiabetics comprise e.g. insulins, amylin, derivatives of GLP-1 and GLP-2 such as, for example, those disclosed in WO 98/08871 and orally active hypoglycemic active ingredients. The orally active hypoglycemic active ingredients preferably comprise sulfonylureas, e.g tolbutamide, glibenclamide, glimepiride, glipizide, gliquidone, glisoxepide, glibomuride or gliclazide; biguanides, e.g. metformin; meglitinides, e.g. repaglinide; beta3 adrenergic agonists; oxadiazolidinediones; glucosidase inhibitors e.g. alphaglucosidase inhibitors such as miglitol or acarbose; glucagon receptor antagonists, GLP-1 agonists, potassium channel openers like diazoxide or those disclosed in WO 97/26265 or WO 99/03861; CB-1 (cannabinoid-1 receptor) antagonists/inverse agonists; insulin sensitizers like thiazolidinediones, e.g. troglitazone, ciglitazone, pioglitazone, rosiglitazone or the compounds disclosed in WO 97/41097, in particular 5-[[4-[(3,4-dihydro-3-methyl-4-oxo-2-quinazolinylmethoxy]pheny-1]methyl]-2,4-thiazolidinedione; activators of insulin receptor kinase; inhibitors of liver enzymes involved in the stimulation of gluconeogenesis and/or glycogenolysis, for example inhibitors of glycogen phosphorylase; and modulators of glucose uptake and glucose excretion.

Suitable antiobesity or appetite-regulating agents comprise one or more of a 5-HT (serotonin) transporter inhibitor, a NE (norepinephrine) transporter inhibitor, a CB-1 (cannabinoid-1 receptor) antagonist/inverse agonist, a ghrelin antibody, a ghrelin antagonist, a H3 (histamine H3) antagonist/inverse agonist, a MCH1R (melanin concentrating hormone 1 R) antagonist, a MCH2R (melanin concentrating hormone 2R) agonist/antagonist, a NPY1 (neuropeptide Y Y1) antagonist, a NPY2 (neuropeptide Y Y2) agonist, a NPY5 (neuropeptide Y Y5) antagonist, leptin, a leptin derivative, an opioid antagonist, an orexin antagonist, a BRS3 (bombesin receptor subtype 3) agonist, a CCK-A (cholecystokinin-A) agonist, a CNTF (ciliary neurotrophic factor), a CNTF derivative, a GHS (growth hormone secretagogue receptor) agonist, SHT2c (serotonin receptor 2c) agonist, a Mc3r (melanocortin 3 receptor) agonist, a Mc4r (melanocortin 4 receptor) agonist, a monoamine reuptake inhibitor, a serotonin reuptake inhibitor, a GLP-1 (glucagon-like peptide 1) agonist, topiramate, phytopharm compound 57, an ACC2 (acetyl-CoA carboxylase-2) inhibitor, a beta3 adrenergic agonist, a DGAT1 (diacylglycerol acyltransferase 1) inhibitor, a DGAT2 (diacylglycerol acyltransferase 2) inhibitor, a FAS (fatty acid synthase) inhibitor, a PDE (phosphodiesterase) inhibitor, a thyroid hormone B agonist, an UCP-1 (uncoupling protein 1), 2, or 3 activator, an acyl-estrogen, a glucocorticoid antagonist, an 11 HSD-1 (11-beta hydroxy steroid dehydrogenase type 1) inhibitor, a SCD-1 (stearoyl-CoA desaturase-1) inhibitor, a dipeptidyl peptidase IV (DP-IV) inhibitor, a lipase inhibitor, a fatty acid transporter inhibitor, a dicarboxylate transporter inhibitor, a glucose transporter inhibitor, a phosphate transporter inhibitor, and pharmaceutically acceptable salts and esters thereof.

Suitable appetite-regulating agents (appetite suppressants) comprise sibutramine or the mono- and bisdemethylated active metabolites of sibutramine; fenfluramine or dexfenfluramine; mazindol, diethylpropion or phentermine; leptin or modified leptin; dexamphetamine and amphetamine.

Suitable lipase inhibitors comprise orlistat, panclicins, lipase inhibitors isolated from micro organisms such as lipstatin (from *Streptomyces toxytricini*), ebelactone B (from *Streptomyces aburaviensis*), synthetic derivatives of these compounds; 2-oxy-4H-3,1-benzoxazin-4-one derivatives like Alizyme's ATL-962 or structurally related compounds; 2-amino-4H-3,1-benzoxazin-4-one derivatives or extracts of plants known to possess lipase inhibitory activity, e.g. extracts of *Alpinia officinarum* or compounds isolated from such extracts like 3-methylethergalangin (from *A. officinarum*);

Suitable $CB_1$-cannabinoid antagonists include rimonabant, SLV319, SR147778 and CP-945598.

Suitable cardiovascular active agents comprise angiotensin II receptor antagonists, e.g. abitesartan, benzyllosartan, candesartan, elisartan, embusartan, enoltasosartan, eprosartan, fonsartan, forasartan, glycyllosartan, irbesartan, isoteoline, losartan, milfasartan, olmesartan, opomisartan, pratosartan, ripisartan, saprisartan, saralasin, sarmesin, tasosartan, telmisartan, valsartan, zolasartan; Kissei KRH-94, Lusofarmaco LR-B/057, Lusofarmaco LR-B/081, Lusofarmaco LR B/087, Searle SC-52458, Sankyo CS-866, Takeda TAK-536, Uriach UR-7247, A-81282, A-81988, BIBR-363, BIBS39, BIBS-222, BMS-180560, BMS-184698, CGP-38560A, CGP-48369, CGP-49870, CGP-63170, CI-996, CV-11194, DA-2079, DE-3489, DMP-811, DuP-167, DuP-532, GA-0056, E-4177, EMD-66397, EMD-73495, EXP-063, EXP-929, EXP-3174, EXP-6155, EXP-6803, EXP-7711, EXP-9270, FK-739, HN-65021, HR-720, ICI-D6888, ICI-D7155, ICI-D8731, KRI-1177, KT3-671, KW-3433, L-158809, L-158978, L-159282, L-159689, L-159874, L-161177, L-162154, L-162234, L-162441, L-163007, L-163017, LY-235656, LY-285434, LY-301875, LY-302289, LY-315995, ME-3221, PD-123177, PD-123319, PD-150304, RG-13647, RWJ-38970, RWJ-46458, S-8307, S-8308, SL-91.0102, U-96849, U-97018, UP-269-6, UP-275-22, WAY-126227, WK-1492.2K, WK-1360, X-6803, XH-148, XR-510, YM-358, YM-31472, ZD-6888, ZD-7155 and ZD-8731 or any physiologically compatible salts, solvates, prodrugs or esters thereof; daglutril; non-selective alpha-adrenoceptor antagonists, e.g. tolazoline or phenoxybenzamine; selective alpha-adrenoceptor antagonists, e.g. doxazosin, prazosin, terazosin or urapidil; beta-adrenoceptor antagonists, e.g. acebutolol, alprenolol, atenolol, betaxolol, bisoprolol, bupranolol, carazolol, carteolol, celiprolol, mepindolol, metipranolol, metoprolol, nadolol, oxprenolol, penbutolol, pindolol, propranolol, sotalol and timolol; mixed antagonists of alpha- and beta-adrenoceptors, e.g. carvedilol or labetolol; ganglion blockers, e.g. reserpine or guanethidine; alpha2-adrenoceptor agonists (including centrally acting alpha2-adrenoceptor agonists), e.g. clonidine, guanfacine, guanabenz methyldopa and moxonidine; renin-inhbitors, e.g. alskiren; ACE-inhbitors, e.g. benazepril, captopril, cilazapril, enalapril, fosinopril, imidapril, lisinopril, moexipril, quinapril, perindopril, ramipril, spirapril or trandolapril; mixed or selective endothelin receptor antagonists e.g. atrasentan, bosentan, clazosentan, darusentan, sitaxsentan, tezosentan, BMS-193884 or J-104132; direct vasodilators, e.g. diazoxide, dihydralazine, hydralazine or minoxidil; mixed ACE/NEP-inhbitors, e.g. omapatrilat; ECE-inhbitors, e.g. FR-901533; PD-069185; CGS-26303; CGS-34043; CGS-35066; CGS-30084; CGS-35066; SM-19712; Ro0677447; selective NEP-inhibitors; vasopressin antagonists, aldosterone receptor antagonists, e.g. eplerenone or spironolactone;

angiotensin vaccine; and urotensin II receptor antagonists.

Suitable diuretics comprise thiazide diuretics, e.g. althiazide, bemetizide, bendroflumethiazide, benzylhydrochlorothiazide, benzthiazide, buthiazide, chlorothiazide, cyclothiazide, hydrochlorothiazide, hydroflumethiazide, methyclothiazide, paraflutizide, polythiazide, teclothiazide, trichlormethiazide; thiazide analogue diuretics, e.g. chloraminofenamide, chlortalidone, clofenamide, clopamide, clorexolone, fenquizone, indapamide, mefruside, metolazone, quinethazone, tripamide, xipamide; loop diuretics, e.g. azosemide, bumetanide, furosemide, piretanide, torsemide; potassium sparing diuretics, e.g. amiloride, potassium canrenoate, spironolactone, triamterene or any physiologically compatible tautomers, salts, solvates, prodrugs or esters of any afore mentioned diuretic.

Suitable active agents which alter lipid levels comprise compounds which alter lipid metabolism, such as antihyperlipidemic active ingredients and antilipidemic active ingredients like HMGCoA reductase inhibitors, e.g. atorvastatin, berivastatin, cerivastatin, crilvastatin, fluvastatin, glenvastatin, lovastatin, mevastatin, pitavastatin, pravastatin, rosuvastatin, simvastatin or any physiologically compatible salts, solvates, prodrugs or esters thereof; inhibitors of cholesterol transport/of cholesterol uptake; inhibitors of bile acid reabsorption or inhibitors of the microsomal triglyceride transfer protein (MTP); compounds which reduce food intake, PPAR (= peroxisome proliferator-activated receptors) and RXR agonists and active agents which act on the ATP-dependent potassium channel of the beta cells; fibric acids, e.g. bezafibrate, ciprofibrate, clofibrate, fenofibrate or gemfibrozil; cholestyramine, colestipol, probucol, ezetimibe and dextrothyroxine; HMGCoA synthase inhibitor, a cholesterol absorption inhibitor, an acyl coenzyme A-cholesterol acyl transferase (ACAT) inhibitor, a cholesteryl ester transfer protein (CETP) inhibitor, a squalene synthetase inhibitor, an antioxidant, a PPAR a agonist, a FXR receptor modulator, a LXR receptor agonist, a lipoprotein synthesis inhibitor, a renin angiotensin system inhibitor, a microsomal triglyceride transport inhibitor, a bile acid reabsorption inhibitor, a PEAR8 agonist, a triglyceride synthesis inhibitor, a transcription modulator, a squalene epoxidase inhibitor, a low density lipoprotein receptor inducer, a platelet aggregation inhibitor, a 5-LO or FLAP inhibitor, a PPAR 8 partial agonist, and niacin or a niacin receptor agonist, and pharmaceutically acceptable salts and esters thereof.

Further active agents which may be suitable for use in combination with the compound of general Formula I according to the present invention may be selected from the group consisting of CART agonists, H3 antagonists, TNF agonists, CRF agonists, CRF BP antagonists, urocortin agonists, beta3-agonists, MSH (melanocyte-stimulating hormone) agonists, serotonin-reuptake inhibitors, mixed serotonin- and noradrenaline-reuptake inhibitors, 5HT modulators, MAO inhibitors, galanin antagonists, growth hormone, growth hormone-releasing compounds, TRH agonists, modulators of uncoupling proteins 2 or 3, leptin agonists, dopamine agonists (bromocriptine, doprexin), RXR modulators, hCNTF agonists and TR-beta-agonists.

Preferred pharmaceutical combination compositions according to the invention comprise combinations of at least one compound of general Formula I and at least one biguanide; at least one compound of general Formula I and at least one fibric acid; at least one compound of general Formula I and at least one HMGCoA reductase inhibitor; and at least one compound of general Formula I and at least one insulin sensitizer.

Preferred compounds of general formula I for combination with one or more of the above mentioned active agents are 4-phenyl-piperazine-1-sulfonic acid amide; 4-(2-chloro-phenyl)-piperazine-1-sulfonic acid amide; 4-(2-methoxy-phenyl)-piperazine-1-sulfonic acid amide; 4-pyridin-4-yl-piperazine-1-sulfonic acid amide; 4-pyrimidin-2-yl-piperazine-1-sulfonic acid amide; 4-(4-fluoro-phenyl)-piperazine-1-sulfonic acid amide; 4-(4-chloro-3-trifluoromethyl-phenyl)-piperazine-1-sulfonic acid amide and/or 4-(3-chloro-5-trifluoromethyl-pyridin-2-yl)-piperazine-1-sulfonic acid amide.

Metformine is the preferred biguanide for combination with at least one compound of general Formula I.

Preferred fibric acids for combination with at least one compound of general Formula I are bezafibrate, ciprofibrate, clofibrate, fenofibrate and/or gemfibrozil. Fenofibrate is most preferred.

Preferred HMGCoA reductase inhibitors for combination with at least one compound of general Formula I are atorvastatin, berivastatin, cerivastatin, crilvastatin, fluvastatin, glenvastatin, lovastatin, mevastatin, pitavastatin, pravastatin, rosuvastatin and/or simvastatin or any physiologically compatible salts, solvates, prodrugs or esters thereof. Most preferred are simvastatin, lovastatin and/or pravastatin.

Preferred insulin sensitizers for combination with at least one compound of general Formula I are thiazolidinediones, in particular troglitazone, ciglitazone, pioglitazone and/or rosiglitazone. Rosiglitazone and pioglitazone are most preferred.

More preferred combinations according to the invention are the combination of 4-phenyl-piperazine-1-sulfonic acid amide with metformine; 4-phenyl-piperazine-1-sulfonic acid amide with fenofibrate; 4-phenyl-piperazine-1-sulfonic acid amide with simvastatin and 4-phenyl-piperazine-1-sulfonic acid amide with rosiglitazone.

In one embodiment of the pharmaceutical combination compositions as described above and according to the invention, the compounds of general Formula I can be obtained and administered together with the different active agents, e.g. in one combined unit dosage form like in one tablet or capsule, i.e. in a physical combination. In such a combined unit dosage form, the compound of general Formula I and the different active agents can be segregated from each other, e.g. by means of different layers in said tablet, e.g. by the use of inert intermediate layers known in the art; or by means of different compartments in said capsule. The corresponding active agents or their pharmaceutically

acceptable salts may also be used in form of their hydrates or include other solvents used for crystallization. A unit dosage form may be a fixed combination. A unit dosage form, in particular a fixed combination of the compound of general Formula I and one or more of the different active agents is a preferred alternative of this embodiment.

In another embodiment the compounds of general Formula I and the different active agents can be obtained and administered in two or more separate unit dosage forms, e.g. in two or more tablets or capsules, the tablets or capsules being physically segregated from each other. The two or more separate unit dosage forms can be administered simultaneously or stepwise (separately), e.g. sequentially one after the other in either order. Thus, the compounds of general Formula I and the different active agents can be administered in either order at the same time or at different times spread over the day, the optimal dosage regimen usually being determined by prescription of a physician.

The following examples are intended to explain the invention further, without limiting its scope.

Example 1:

Reaction between 5-Bromo-2-hydroxy acetophenone with N-Boc-4-piperidone

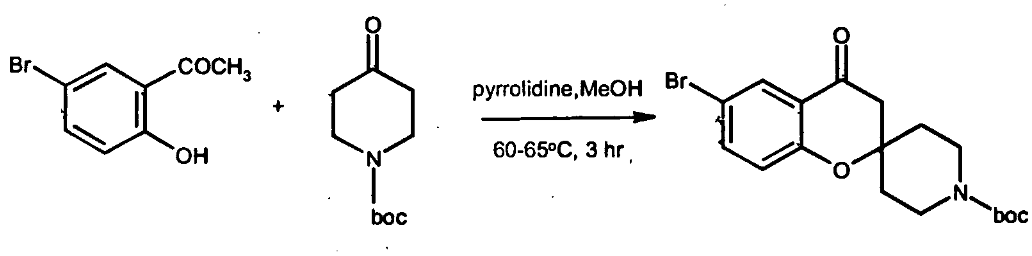


In a 500 ml 3-necked flask, fitted with a reflux condenser, a $CaCl_2$ gaurd tube and a magnetic needle, 0.1 mol N-Boc-4-piperidone (19.5 g) and 0.116 mol pyrrolidine (8.95 g), dissolved in 100 ml of anhydrous MeOH at ambient temperature were added to 0.0837 mol 5-Bromo-2-hydroxyacetophenone (18 g). The reaction mixture was stirred under reflux at 60-65°C for about 3 hrs. The reaction mixture was transferred to a rotary flask and MeOH was distilled off to obtain an orange viscous liquid. 75 ml of water was added and the product was extracted with ethyl acetate (4 times 100 ml). The aqueous layer was discarded and the organic layer was dried over anhydrous $Na_2SO_4$. Ethyl acetate was distilled off and an orange viscous oil was obtained that was thoroughly dried in vacuo. Further purification by column chromatography with ethyl acetate/petrolether 10/90 yielded 28,7 g (87%) of a pale yellow crystalline solid. Melting Point: 140 -143°C.

Table 3: Further compounds (X-Y is $H_2C$-C=O) prepared in excellent yields by following the same manner as in example 1.

| R3 | R4 | R5 | R6 | m. p. / °C |
|---|---|---|---|---|
| H | H | H | H | 89-92 |
| H | Br | H | H | 140-143 |
| H | OCH3 | H | H | 146-149 |
| H | F | H | H | 118-122 |
| H | H | F | H | 104-107 |
| H | NH2 | H | H | 183-185 |
| H | CN | H | H | 178-181 |
| H | H | NH-COCF3 | H | 194-196 |
| H | NH-COCF3 | H | H | 230-232 |
| H | Cl | H | Br | 126-128 |
| H | H | Br | H | 112-115 |

Example 2:

Protection group bond cleavage

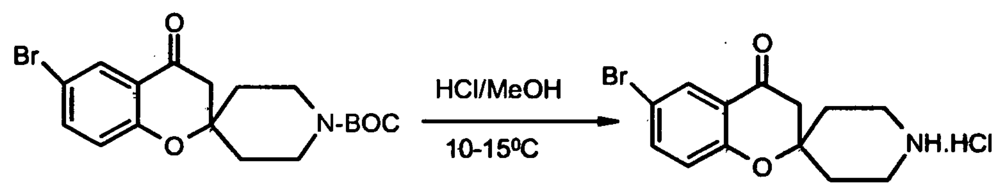


In a 1 l 3-necked flask, fitted with a bubbler, a magnetic needle and a set up to pass HCl gas, 26.0 g of 5-Bromo-N-bocspirocyclic ether derivative was dissolved in 300 ml methanol. HCl gas was passed through the solution until a white solid started precipitating out. This indicated the completion of the reaction and saturation of HCl in the reaction mixture. The reaction mixture was transferred to a rotary flask and methanol was removed under vacuum. A pale yellow solid was obtained, which was washed well with petrol ether, filtered through a Bucker's funnel and thoroughly dried in vacuo. 22 g HCl salt was obtained as an off white crystalline solid. Melting Point: 273 - 274˚C

Table 4: Further compounds (X-Y is $H_2C-C=O$) prepared in excellent yields by following the same manner as in example 2.

| R3 | R4 | R5 | R6 | M. P. / °C |
|---|---|---|---|---|
| H | H | H | H | 206-207 |
| H | Br | H | H | 273-274 |
| H | OCH3 | H | H | 199-202 |
| H | F | H | H | 223-225 |
| H | H | F | H | 200-206 |
| H | NH2 | H | H | 211-220 |
| H | CN | H | H | 131-135 |
| H | Cl | H | Br | 133-134 |
| H | H | Br | H | 222-228 |

Example 3:

Preparation of 5-Bromo-spirocyclic ether sulfamide derivative

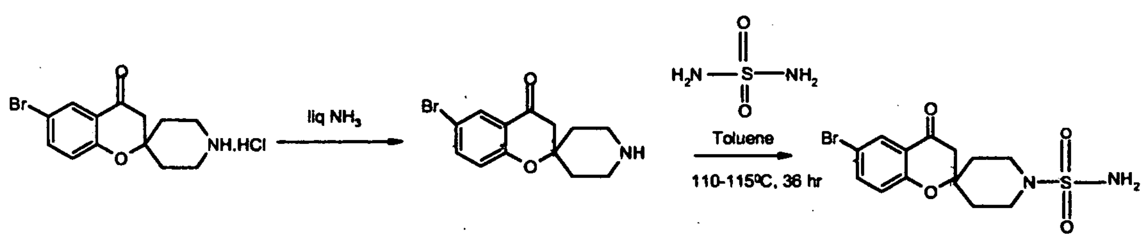


16 g of the 5-Bromo HCl salt from Example 2 were added to 250 ml of water and neutralized by adding aqueous $NH_3$ until pH = -10. A yellow sticky paste was formed which was extracted with ethyl acetate (4 times 100ml). The organic

layer was then dried over anhydrous sodium sulphate and finally concentrated using a rotary evaporator to get the free base as dark yellowish orange viscous oil. The free base was thoroughly dried under vacuum to remove any traces of ethyl acetate. The free base was then dissolved in toluene and solid sulfamide was added. The reaction mixture was stirred under reflux at 110-115°C for about 14 hrs. TLC was checked at regular intervals. There were still spots of starting material. Thus 0.2 equivalent of sulfamide was again added and the reaction mixture was stirred under reflux overnight.

A sticky solid formed during the reaction was removed by dissolving in DCM-MeOH mixture. The toluene solvent was decanted off in to a rotary flask. Some of the solid which did not dissolve in DCM-MeOH or any other solvent was dissolved in DMF (~ 10-15 ml). Half of the solvent was distilled off using a rotary evaporator. The entire quantity was not distilled off as the product was difficultly soluble in any solvent. Hence, in the remaining quantity of solvent, silica (mesh 230-400) was added to it and a slurry was made as such for column chromatography, starting with DCM first and then changing to DCM:MeOH 95 : 5. The obtained pale yellow solid was crystallized using DCM:MeOH:Petrolether, yield of 8.2 g of a white crystalline solid with a melting point of 202-205°C.

The diamino equivalents (R1, R2 = hydrogen) of compounds 1 to 6 and 8 to 78 are prepared in excellent yields by following the same manner as described in the examples 1 to 3 above.

Table 5: Further compounds of general Formula I (X is $CH_2$ and Y is C=O).

| Ex. No. | R1 | R2 | R3 | R4 | R5 | R6 | m.p. / °C |
|---|---|---|---|---|---|---|---|
| 1 | H | H | H | H | H | H | 172-174 |
| 2 | H | H | H | Br | H | H | 205-208 |
| 3 | H | H | H | $OCH_3$ | H | H | 172-177 |
| 4 | H | H | H | Cl | H | H | |
| 5 | H | H | H | F | H | H | 177-186 |
| 6 | H | H | H | H | F | H | 163-166 |
| 7 | H | H | H | $CH_3$ | $CH_3$ | H | |
| 8 | H | H | H | $CH_3$ | H | H | |
| 9 | H | H | H | H | 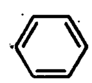 | | |
| 10 | H | H | H | H | $OCH_3$ | H | |
| 11 | H | H | $OCH_3$ | H | H | H | |
| 12 | H | H | H | H | $OCH_2C_6H_5$ | $CH_3$ | |
| 13 | H | H | 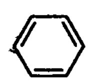 | | H | H | |
| 14 | H | H | H | Cl | H | Cl | |
| 15 | H | H | H | H | $OCH_3$ | $OCH_3$ | |
| 16 | H | H | H | $OCH_3$ | $OCH_3$ | H | |
| 17 | H | H | $OCH_3$ | $OCH_3$ | $OCH_3$ | H | |
| 18 | H | H | $OCH_3$ | H | $OCH_3$ | H | |
| 19 | H | H | $OCH_3$ | H | $OCH_3$ | $CH_3$ | |
| 20 | H | H | H | H | $COCH_3$ | H | |
| 21 | H | H | H | F | H | F | |
| 22 | H | H | $OCH_3$ | 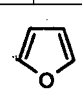 | | $OCH_3$ | |
| 23 | H | H | H | $C_2H_5$ | H | H | |
| 24 | H | H | H | H | $OCOCH_3$ | H | |

(continued)

| Ex. No. | R1 | R2 | R3 | R4 | R5 | R6 | m.p. / °C |
|---|---|---|---|---|---|---|---|
| 25 | H | H | H | H | 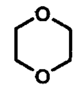 | | |
| 26 | H | H | H | $NH_2$ | H | H | |
| 27 | H | H | H | H | $HNC_2H_5$ | $C_3H_7$ | |
| 28 | H | H | H | C≡N | H | H | |
| 29 | H | H | H | H | $HNCOCF_3$ | $C_3H_7$ | |
| 30 | H | H | H | tert. $4C_4H_9$ | H | tert. $C_4H_9$ | |
| 31 | H | H | $OCH_3$ | H | H | $OCH_3$ | |
| 32 | H | H | H | $HNCOCF_3$ | H | H | |
| 33 | H | H | H | H | H | Cl | |
| 34 | H | H | H | H | $CH_3$ | H | |
| 35 | H | H | H | H | $HNCOCH_3$ | $CH_2CH =CH_2$ | |
| 36 | H | H | H | H | $OCH_2COC H_3$ | H | |
| 37 | H | H | $OC_2H_4OC_2 H_5$ | H | H | H | |
| 38 | H | H | $OCH_2C_6H_5$ | H | H | H | |
| 39 | H | H | $OCH_2CH_2C H (CH_3)_2$ | H | H | H | |
| 40 | H | H | H | $OC_2H_5$ | H | H | |
| 41 | H | H | H | H | $OCH_3$ | $OCH_2 C_6H_5$ | |
| 42 | H | H | H | $CH=CHCH_3$ | $HNCOCH_3$ | $C_3H_7$ | |
| 43 | H | H | H | F | H | H | |
| 44 | H | H | H | $CH_3$ | 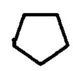 | | |
| 45 | H | H | H | Cl | $CH_3$ | H | |
| 46 | H | H | H | Br | H | Br | |
| 47 | H | H | H | $CH_3$ | H | $NO_2$ | |
| 48 | H | H | H | C≡N | C! | Br | |
| 49 | H | H | Cl | H | H | H | |
| 50 | H | H | H | H | $NO_2$ | H | |
| 51 | H | H | H | H | Cl | H | |
| 52 | H | H | 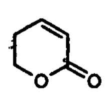 | | H | H | |
| 53 | H | H | H | F | H | Cl | |
| 54 | H | H | H | $OCH_3$ | $CH_3$ | H | |
| 55 | H | H | H | Cl | H | Br | 192-195 |
| 56 | H | H | H | $iC_3H_7$ | H | H | |
| 57 | H | H | F | H | H | H | |

(continued)

| Ex. No. | R1 | R2 | R3 | R4 | R5 | R6 | m.p. / °C |
|---|---|---|---|---|---|---|---|
| 58 | H | H | H | $OCF_3$ | H | H | |
| 59 | H | H | H | $CH_3$ | H | $CH_3$ | |
| 60 | H | H | H | Br | H | Cl | |
| 61 | H | H | $OCH_3$ | H | $OCH_3$ | I | |
| 62 | H | H | H | $CH_3$ | H | HN-COO-tert.- $C_4H_9$ | |
| 63 | H | H | H | F | H | 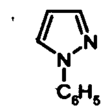 | |
| 64 | H | H | H | F | H | 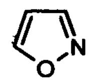 | |
| 65 | H | H | H | Cl | $OCH_3$ | H | |
| 66 | H | H | H | $NSO_2CH_3$ | H | H | |
| 67 | H | H | H | H | Br | H | 186-188 |
| 68 | H | H | $OCH_3$ | H | $OCH_3$ $OCH_2$-3- | Cl | |
| 69 | H | H | H | H | $C_6H_4CF_3$ | H | |
| 70 | H | H | $OCH_2CH_3$ | H | H | H | |
| 71 | H | H | H | $OCH_2CH_3$ | H | H | |
| 72 | H | H | H | 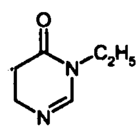 | | H | |
| 73 | H | H | H | Cl | 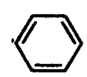 | | |
| 74 | H | H | H | $OCH_3$ | 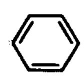 | | |
| 75 | H | H | H | H | 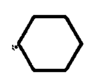 | | |
| 76 | H | H | H | 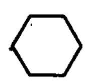 | | H | |
| 77 | H | H | H | $COOCH_3$ | H | H | |

Example 4

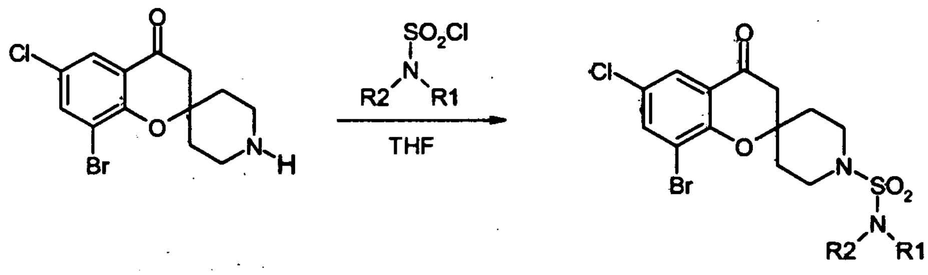


1 equivalent of benzopyrane derivative in THF and 1 equivalent of sulfamoylchloride derivative in THF were reacted at room temperature over night in the presence of 1.1 equivalent of triethylamine. The reaction mixture was evaporated and purified by liquid/liquid extraction with 10% tartatic acid and ethyl acetate. The organic layers were dried on sodium carbonate and evaporated. The LC-MS data are summarized below.

Table 6: Further compounds of general Formula I (X is $CH_2$ and Y is C=O) prepared in excellent yields by following the same manner as in example 4.

| Example No | Structure | Molweight | ELSD Retention time / min |
|---|---|---|---|
| 78 | 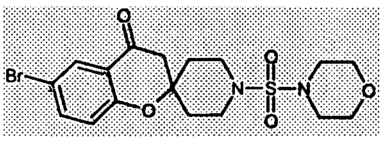 | 445,3319 | 5,62 |
| 79 | 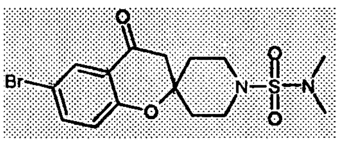 | 403,2951 | 5,72 |
| 80 | 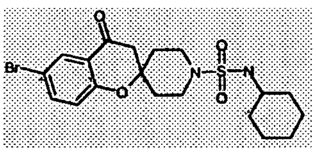 | 457,3865 | 5,85 |
| 81 | 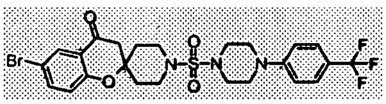 | 588,4425 | 6,78 |
| 82 | 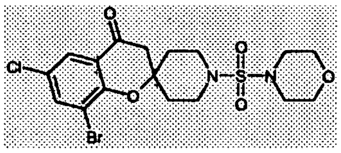 | 479,777 | 5,87 |
| 83 | 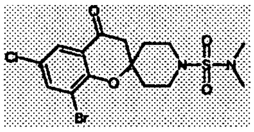 | 437,7402 | 6,01 |
| 84 | 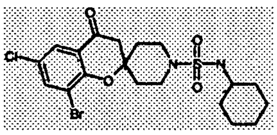 | 491,8316 | 6,51 |

(continued)

| Example No | Structure | Molweight | ELSD Retention time / min |
|---|---|---|---|
| 85 | 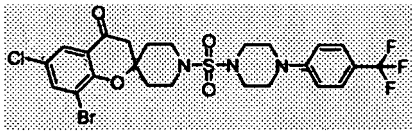 | 622,8876 | 6,95 |
| 86 | 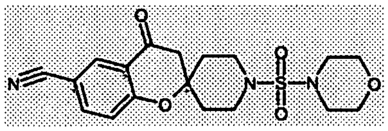 | 391,4459 | 5 |
| 87 | 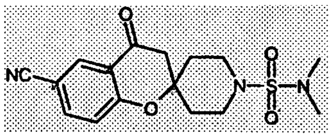 | 349,4091 | 5,1 |
| 88 | 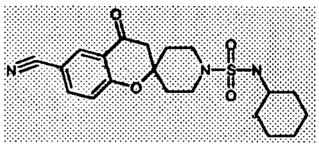 | 403,5005 | 5,68 |
| 89 | 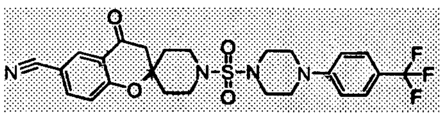 | 534,5565 | 6,33 |
| 90 | 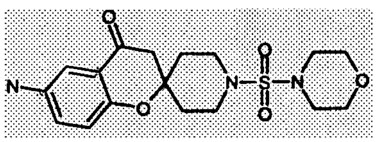 | 381,4507 | 4,35 |

Example 5

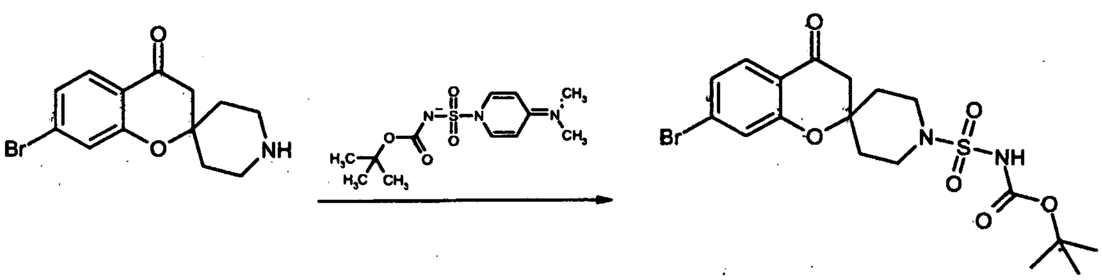

A mixture of 1.2 g benzopyran, 0.6 ml of triethyl amine and 1.2 g DMAP reagent III in 10 ml dichloromethane was stirred at room temperature for 4 hours. 37 % hydrochloric acid was added dropwise and the resulting mixture was stirred for 30 minutes. The organic layer was washed with water several times, dried over $Na_2SO_4$ and evaporated. Yield: 0.89 g. LC-MS: ELSD at 5.96 min. Protection group bond cleavage was preformed as in Example 2.

Example 6

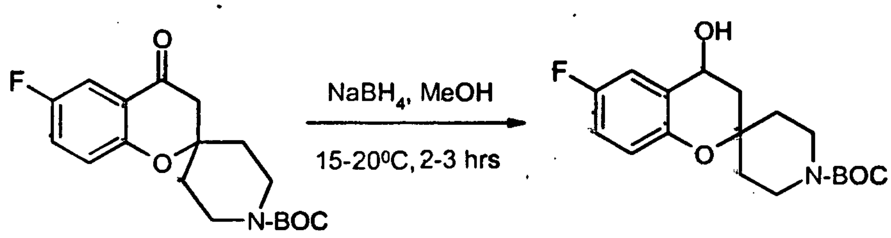


In a three necked 500 ml flask equipped with stirring bar and bubbler, 29 g of the pyranon derivative were dissolved in 250 ml methanol. The reaction mixture was cooled to 10˚C and sodium borohydride (6.6 g) was added portion wise in D.5 hr. One hour later the solvent was removed under vacuum. The solid was taken in water (400 ml) and the product was extracted with ethyl acetate. The organic layer was finally washed with brine solution (250 ml) and dried over anhydrous sodium sulphate. The solvent was concentrated to get 30 g of a pale yellow oily product. The sulfamyl-/sulfamide group substitution was preformed as any of Examples 3 and/or 4.

Example 7

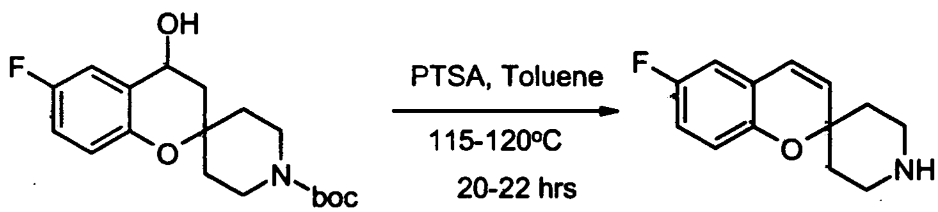


Using a 500 ml three necked flask equipped with a water condenser, stirring bar, guard tube the above synthesized hydroxyl compound (21.0 gm) was dissolved in toluene (350 ml) and refluxed for 10 h together with PTSA (12.9 gm). Toluene was completely removed under vacuum and dichloromethane (400 ml) was added to the residue and stirred to get clear solution. Dichloromethane solution was washed with 10 % $NaHCO_3$(2 x 100ml) and then with saturated salt solution (200 ml). The organic layer was dried over anhydrous sodium sulphate. The dichloromethane layer was concentrated to get 31 g of crude product. Finally the crude product was purified using flash column chromatography over silica gel (230-400 mesh) using dichloromethane (94%) : methanol (5%): ammonia (1%) as elution system. Yield: 13 g of a colourless solid.

Table 7: Further compounds of general Formula I prepared in excellent yields by following the same manner as in example 7.

| R3 | R4 | R5 | R6 | m.p./˚C |
|---|---|---|---|---|
| H | OCH3 | H | H | 238-240 |
| H | Br | H | H | 258-260 |
| H | F | H | H | 218-220 |

The sulfamyl-/sulfamide group introduction was preformed as any of Examples 3 and/or 4.

Example 8

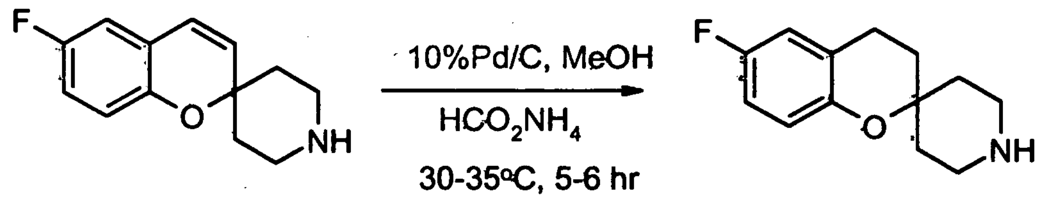


5.0 g of 10% Pd/C was stirred under $N_2$ atmosphere in a 1 litre three necked flask equipped with $N_2$-adaptor and bubbler. Under cooling methanol (200 ml) was added and subsequently the fluoro derivative (17 g) of Example 7 dissolved in methanol (150 ml) was charged directly in the reaction flask. After 15 min of stirring, ammonium formate (17.4 gm) was charged portion wise at ambient temperature. After complete addition, reaction was warmed to 30-35°C and maintained for 5-6 hrs. The catalyst was filtered over high-flow bed and the filtrate was concentrated to get 21 g of the solid product. To this solid, 20% $K_2CO_3$ solution (100 ml) was added and product was extracted by ethyl acetate (3 x 100 ml). All organic layers were combined and washed with saturated solution (200 ml) and dried over anhydrous sodium sulphate. The solvent was evaporated completely to get the product, which was converted to its MCl salt (colourless solid, 14,5 g, mp. 218-220°C).

The sulfamyl-/sulfamide group introduction was preformed as any of Examples 3 and/or 4.

Example 9

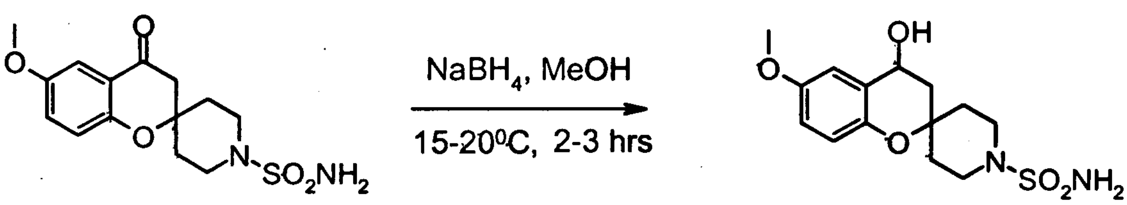


50 mg of the sulfamide were dissolved in 5 ml methanol. The reaction mixture was cooled to 10°C and sodium borohydride was added portion wise in 0.5 hr. One hour later the solvent was removed under vacuum. Water was added to the solid residue and the product was extracted with ethyl acetate. The organic layer was finally washed with brine solution and dried over anhydrous sodium sulphate. The solvent was concentrated to get 43,5 mg of a oily product.

Example 10

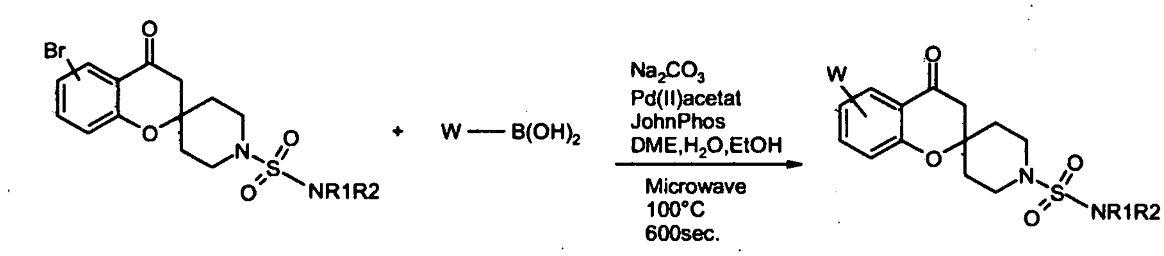


Pd-acetate (0.01 mmol), 0.01 mmol of 2-(Di-tert-butylphosphino)biphenyl ["JohnPhos"] and 0.3 mmol of $Na_2CO_3$ are stirred together with 2 ml of the solvent mixture (DME/$H_2O$/EtOH: 7/3/2) for 5 minutes. Subsequently 0.9 mmol of the bromo-substituted sulfamide and 0.1 mmol of the boronic acid in 3 ml of the same solvent mixture are added. The microwave vial is capped and heated for 600 sec at 100° C. After the reaction, the mixture was filtered over Celite, concentrated and analyzed by LC-MS.

Table 8: Further compounds of general Formula I (X is $CH_2$ and Y is C=O) prepared in excellent yields by following the same manner as in example 10.

| Example No | Structure | ELSD Retention time/ min |
|---|---|---|
| 91 | 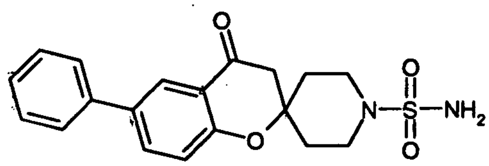 | 5,40 |
| 92 | 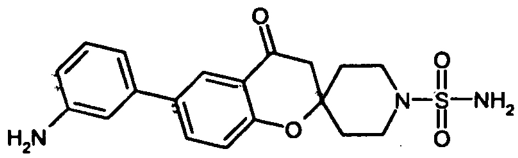 | 4,60 |
| 93 | 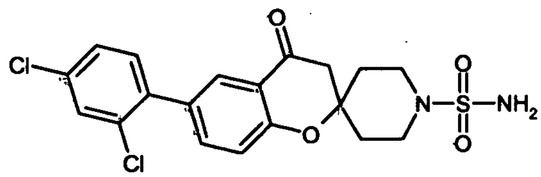 | 6,10 |
| 94 | 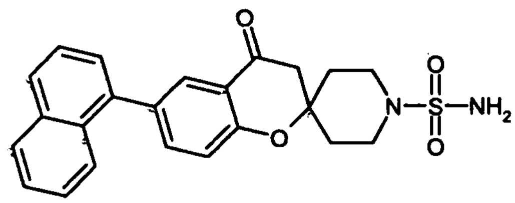 | 5,94 |
| 95 | 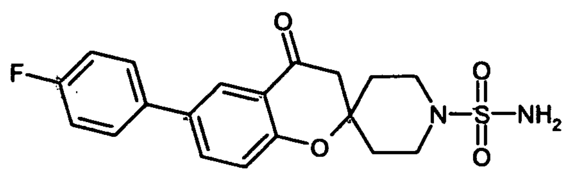 | 5,44 |
| 96 | 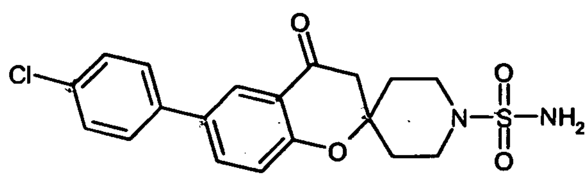 | 5,81 |
| 97 | 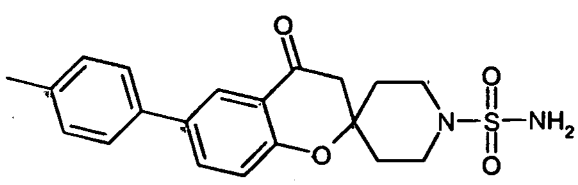 | 5,71 |

(continued)

| Example No | Structure | ELSD Retention time/ min |
|---|---|---|
| 98 | 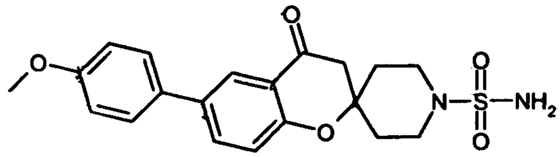 | 5,31 |
| 99 | 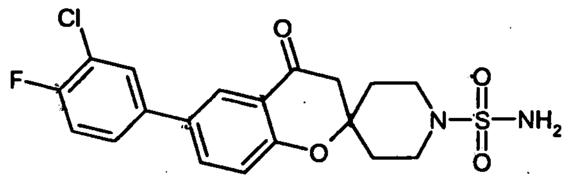 | 5,82 |
| 100 | 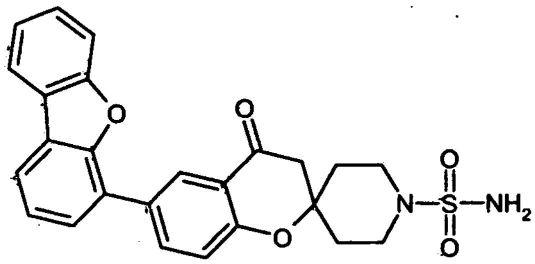 | 6,22 |
| 101 | 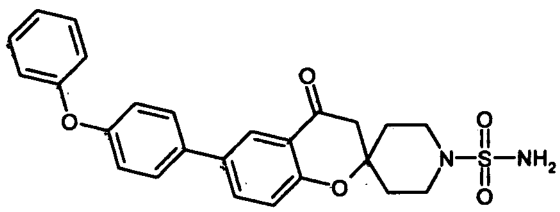 | 6.20 |
| 102 | 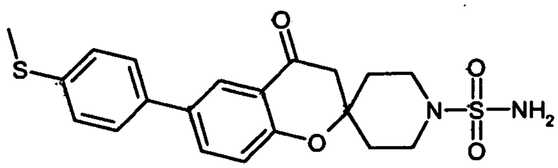 | 5,72 |
| 103 | 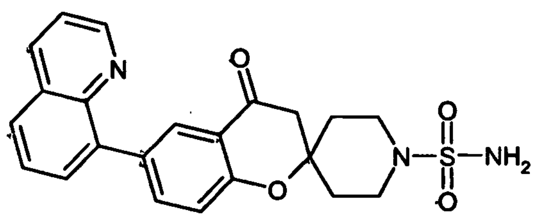 | 5,19 |
| 104 | 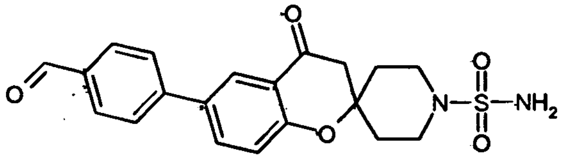 | 4,94 |

(continued)

| Example No | Structure | ELSD Retention time/ min |
|---|---|---|
| 105 | 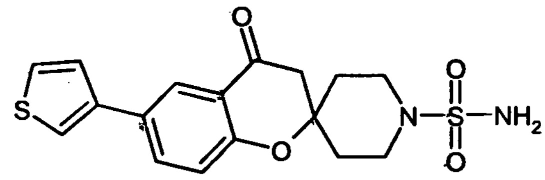 | 5,23 |
| 106 | 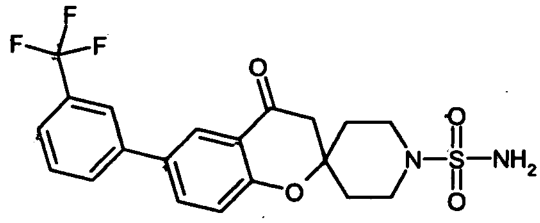 | 5,86 |
| 107 | 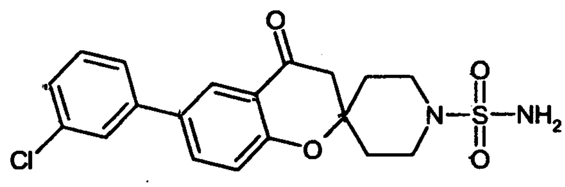 | 5,77 |
| 108 | 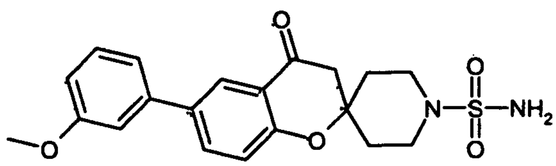 | 5,36 |
| 109 | 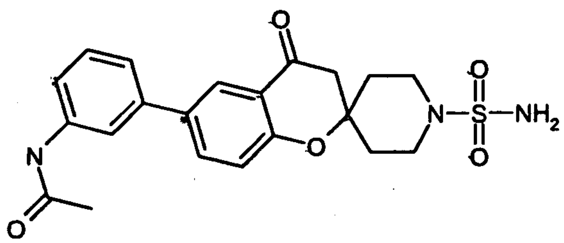 | 4,42 |
| 110 | 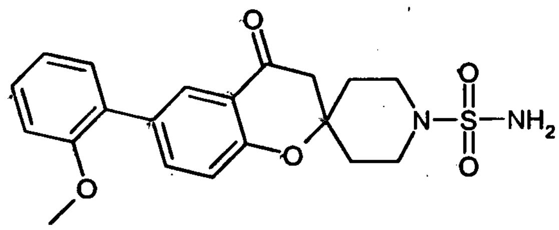 | 5,36 |
| 111 | 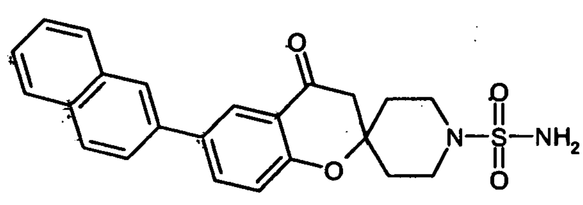 | 5,97 |

(continued)

| Example No | Structure | ELSD Retention time/ min |
|---|---|---|
| 112 | 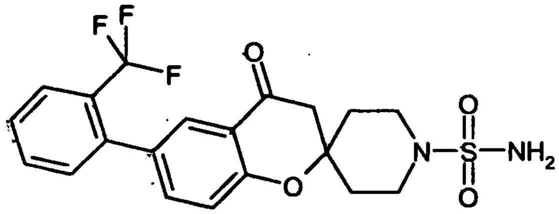 | 5,66 |
| 113 | 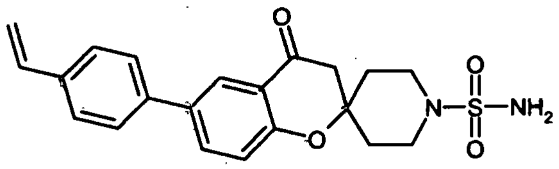 | 5,80 |
| 114 | 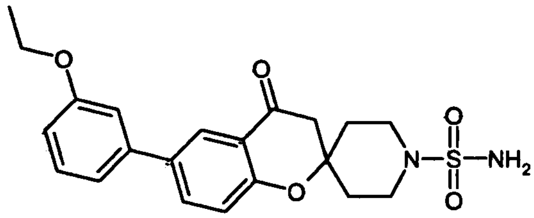 | 5,67 |
| 115 | 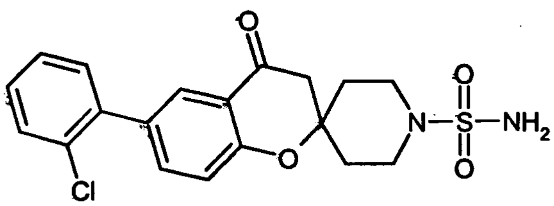 | 5,57 |
| 116 | 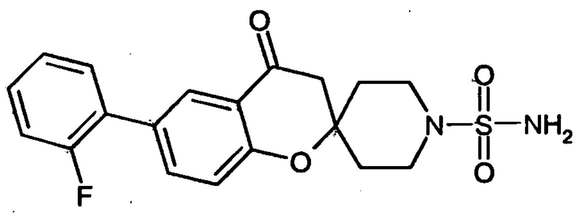 | 5,39 |
| 117 | 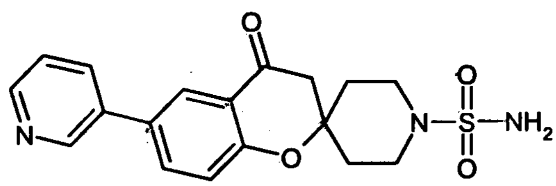 | 4,17 |
| 118 | 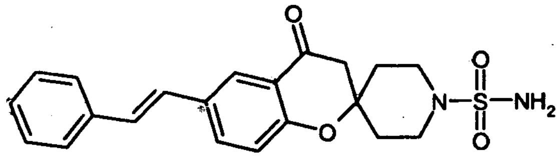 | 5.76 |

(continued)

| Example No | Structure | ELSD Retention time/ min |
|---|---|---|
| 119 | 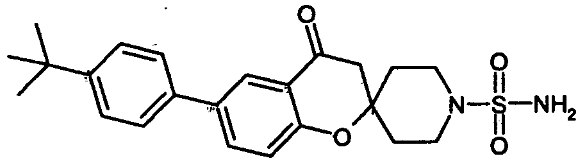 | 6,43 |
| 120 | 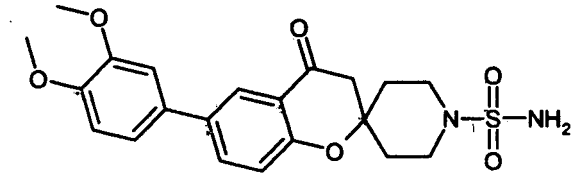 | 4,91 |
| 121 | 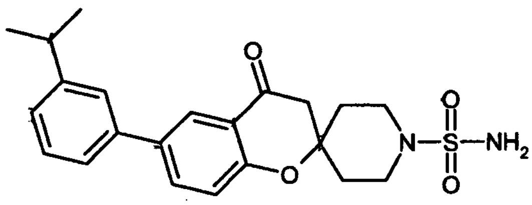 | 6,20 |
| 122 | 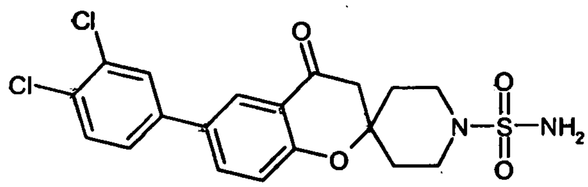 | 6,14 |
| 123 | 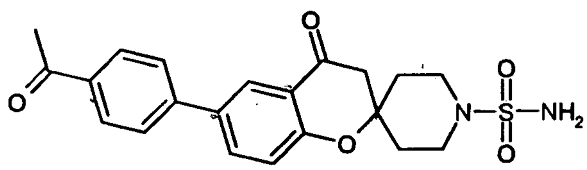 | 4,93 |
| 124 | 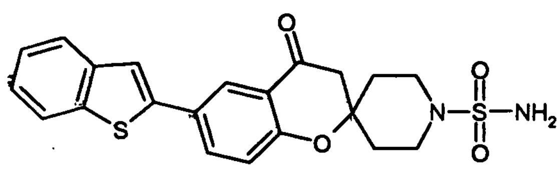 | 6,02 |
| 125 | 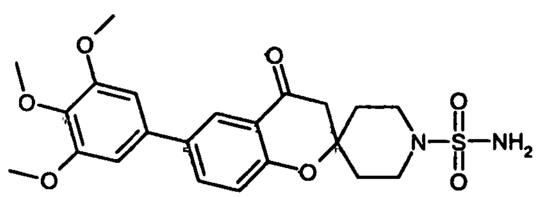 | 4,96 |

(continued)

| Example No | Structure | ELSD Retention time/ min |
|---|---|---|
| 126 | 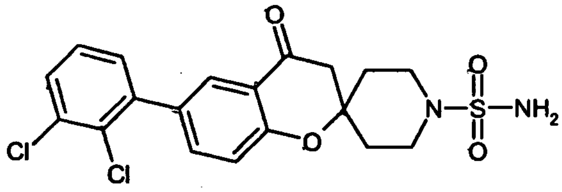 | 5,92 |
| 127 | 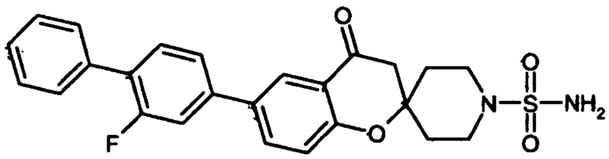 | 6,27 |
| 128 | 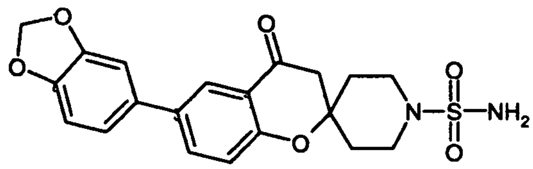 | 5,23 |

Example I:

Capsules containing Compound 1

| | |
|---|---|
| Compound 1 | 70 mg |
| Corn starch | 60 mg |
| Lactose | 250 mg |
| Ethylacetate (= EA) | q.s. |

The active substance, the corn starch and the lactose are processed into a homogeneous pasty mixture using EA. The paste is ground and the resulting granules are placed on a suitable tray and dried at 45°C in order to remove the solvent. The dried granules are passed through a crusher and mixed in a mixer with the further following auxiliaries:

| | |
|---|---|
| Talcum | 5 mg |
| Magnesium stearate | 5 mg |
| Corn starch | 10 mg |

and are then poured into 400 mg capsules (= capsule size 0).

Example II:

Capsules containing Compound 86

| | |
|---|---|
| Compound 86 | 70 mg |
| Corn starch | 60 mg |
| Lactose | 250 mg |
| Ethylacetate (= EA) | q.s. |

The active substance, the corn starch and the lactose are processed into a homogeneous pasty mixture using EA. The paste is ground and the resulting granules are placed on a suitable tray and dried at 45°C in order to remove the solvent. The dried granules are passed through a crusher and mixed in a mixer with the further following auxiliaries:

| | |
|---|---|
| Talcum | 5 mg |
| Magnesium stearate | 5 mg |
| Corn starch | 10 mg |

and are then poured into 400 mg capsules (= capsule size 0).

## Claims

1. A compound of Formula I,

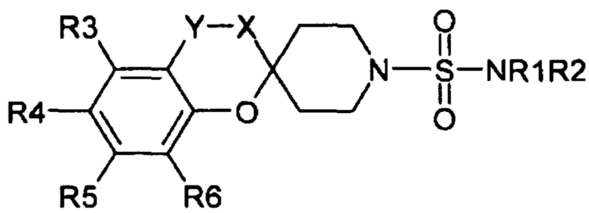

I

wherein
R1 and R2 are independently selected from the group consisting of: hydrogen, $C_1$ to $C_4$ alkyl, $C_4$ to $C_7$ cycloalkyl; or;
R1 and R2 together form a 5 or 6-membered ring which optionally may contain from 1 to 2 heteroatoms independently selected from the group consisting of nitrogen and/or oxygen atoms and which also may be substituted by optionally substituted aryl, optionally substituted heteroaryl or arylenehalogenalkyl;
R3 to R6 are independently selected from the group consisting of: hydrogen; halogen; $C_1$ to $C_4$ alkyl; $C_2$ to $C_4$ alkenyl, optionally substituted with aryl; $C_1$ to $C_4$ alkoxy; $C_1$ to $C_4$ alkoxy substituted with halogen provided that the alpha-carbon atom is substituted by no other halogen than fluorine if any; $C_2$ to $C_4$ alkinyl; $C_1$ to $C_4$ $NSO_2$alkyl; $NH_2$; $NO_2$; $C_1$ to $C_4$ aminoalkyl; $C_2$ to $C_8$ aminodialkyl; cyano; oxyaryl; oxyalkylenearyl; oxyarylenealkyl; oxyalkylene-arylenealkoxy; $C_2$ to $C_8$ ester; $C_1$ to $C_6$ amido; $C_2$ to $C_8$ oxyalkylenecarbonylalkyl; $C_2$ to $C_8$ oxyalkyleneoxyalkyl; $C_1$ to $C_4$ amidooxyalkyl; aryl, optionally substituted; heteroaryl, optionally substituted; condensed aryl, optionally substituted; condensed heteraryl, optionally substituted; or;
R3 and R6 have the same meaning as above and wherein R4 and R5 together form a 5 or 6-membered ring which may optionally contain from 1 to 3 heteroatoms independently selected from the group consisting of: nitrogen, oxygen and sulfur and which may optionally bear 1 or 2 double bonds, and which also may contain a carbonyl group and which also may be substituted by $C_1$ to $C_4$ alkyl, $C_1$ to $C_4$ halogenalkyl, optionally substituted aryl, and/or optionally substituted heteroaryl; or;
R5 and R6 have the same meaning as above and wherein R3 and R4 together form a 5 or 6-membered ring which may optionally contain from 1 to 3 heteroatoms independently selected from the group consisting of: nitrogen, oxygen and sulfur and which may optionally bear 1 or 2 double bonds, and which also may contain a carbonyl group and which also may be substituted by $C_1$ to $C_4$ alkyl, $C_1$ to $C_4$ halogenalkyl, optionally substituted aryl, and/or optionally substituted heteroaryl; or;
R3 and R4 have the same meaning as above and wherein R5 and R6 together form a 5 or 6-membered ring which may optionally contain from 1 to 3 heteroatoms independently selected from the group consisting of: nitrogen, oxygen and sulfur and which may optionally bear 1 or 2 double bonds, and which also may contain a carbonyl group and which also may be substituted by $C_1$ to $C_4$ alkyl, $C_1$ to $C_4$ halogenalkyl, optionally substituted aryl, and/or optionally substituted heteroaryl;
Y-X is selected from the group consisting of: HC=CH, $CH_2$-$CH_2$, O=C-$CH_2$, and (HO)(H)C-$CH_2$;
and its physiologically acceptable acid addition salt.

2. The compound according to Claim 1 wherein
R1 and R2 are independently selected from the group consisting of: hydrogen, $C_1$ to $C_4$ alkyl, $C_4$ to $C_7$ cycloalkyl, or,

R1 and R2 together form a 5 or 6-membered ring which optionally may contain from 1 to 2 heteroatoms independently selected from the group consisting of nitrogen and/or oxygen atoms and which also may be substituted by optionally substituted aryl, optionally substituted heteroaryl, or arylenehalogenalkyl;
R3 to R6 are independently selected from the group consisting of: hydrogen, flourine, chlorine, bromine, methyl, ethyl, propyl, butyl, ethylene, propylene, methoxy, ethoxy, propoxy, ethinyl, propinyl, butinyl, $NSO_2CH_3$, $NH_2$, $NO_2$, aminomethyl, aminoethyl, aminopropyl, aminobutyl, aminodimethyl, aminodiethyl, aminodipropyl, aminodibutyl, cyano, oxyphenyl, oxybenzyl, oxyethylenephenyl, oxyphenylenemethyl, oxyphenylenemethoxy, acetyl, amidomethyl, amidoethyl, oxymethylenecarbonylmethyl, oxyethylenecarbonylmethyl, oxymethylenecarbonylethyl, oxyethylenecarbonylethyl, oxymethyleneoxymethyl, oxymethyleneoxyethyl, oxyethyleneoxymethyl, oxyethyleneoxyethyl, amidooxymethyl, and arnidooxyethyl; and
Y-X is selected from the group consisting of: HC=CH, $CH_2$-$CH_2$, O=C-$CH_2$, and (HO)(H)C-$CH_2$.

**3.** The compound according to any of Claims 1 or 2 wherein
R1 and R2 are both H;
R3 to R6 are independently selected from the group consisting of: hydrogen, halogen and $C_1$ to $C_4$ alkoxy; and
Y-X is O=C-$CH_2$.

**4.** The compound according to any of Claims 1 to 3 wherein
R4 is selected from the group consisting of: hydrogen, chlorine, bromine, and methoxy; and
R5 is selected from the group consisting of: hydrogen and bromine.

**5.** The compound according to Claim 1 wherein
R1 and R2 are independently selected from the group consisting of: hydrogen, $C_1$ to $C_4$ alkyl, $C_4$ to $C_7$ cycloalkyl, or, R1 and R2 together form a 5 or 6-membered ring which optionally may contain from 1 to 2 heteroatoms independently selected from the group consisting of nitrogen and/or oxygen atoms and which also may be substituted by optionally substituted aryl, optionally substituted heteroaryl or arylenehalogenalkyl;
R3, R5 and R6 are independently selected from the group consisting of: hydrogen; halogen; $C_1$ to $C_4$ alkyl; $C_2$ to $C_4$ alkenyl; $C_1$ to $C_4$ alkoxy; $C_1$ to $C_4$ alkoxy substituted with halogen provided that the alpha-carbon atom is substituted by no other halogen than fluorine if any; $C_2$ to $C_4$ alkinyl; $C_1$ to $C_4$ $NSO_2$alkyl; $NH_2$; $NO_2$; $C_1$ to $C_4$ aminoalkyl; $C_2$ to $C_8$ aminodialkyl; cyano; oxyaryl; oxyalkylenearyl; oxyarylenealkyl; oxyalkylenearylenealkoxy; $C_2$ to $C_8$ ester; $C_1$ to $C_8$ amido; $C_2$ to $C_8$ oxyalkylenecarbonylalkyl; $C_2$ to $C_8$ oxyalkyleneoxyalkyl; and; $C_1$ to $C_4$ amidooxyalkyl;
R4 is selected from the group consisting of:

(a) phenyl, optionally substituted with one to three substituents independently selected from the group consisting of: hydrogen, amino, halogen, $C_1$ to $C_4$ alkyl, $C_1$ to $C_4$ alkoxy, oxyaryl, $C_1$ to $C_4$ mercapto, C(O)H, trifluormethyl, -N-C(O)$C_{1-4}$alkyl, $C_2$ to $C_4$ alkenyl, C(O)$C_{1-4}$alkyl, and aryl;
(b) 1-naphthyl, 2-naphthyl, 2-pyridyl, 3-pyridyl, 4-pyridyl, 2-thiophenyl, 3-thiophenyl, quinoline, isoquinoline, benzo[b]thiophene, 1,3-dihydrobenzo[c]thiophene, 1-dibenzofuran, 2-dibenzofuran, 3-dibenzofuran, and, 4-dibenzofuran, each optionally substituted with one to three substituents selected from the group consisting of: hydrogen, amino, halogen, $C_1$ to $C_4$ alkyl, $C_1$ to $C_4$ alkoxy, oxyaryl, $C_1$ to $C_4$ mercapto, C(O)H, trifluormethyl, -N-C(O)$C_{1-4}$alkyl, $C_2$ to $C_4$ alkenyl, C(O)$C_{1-4}$alkyl, and aryl; and;

Y-X is selected from the group consisting of: HC=CH, $CH_2$-$CH_2$, O=C-$CH_2$, and (HO)(H)C-$CH_2$.

**6.** The compound according to Claim 5 wherein
R1, R2, R3, R5 and R6 are each hydrogen; and
R4 is selected from the group consisting of:

(a) phenyl, optionally substituted with one to three substituents independently selected from the group consisting of: hydrogen, amino, halogen, $C_1$ to $C_4$ alkyl, $C_1$ to $C_4$ alkoxy, oxyaryl, C1 to C4 mercapto, C(O)H, trifluormethyl, - N-C(O)$C_{1-4}$alkyl, C2 to C4 alkenyl, C(O)$C_{1-4}$alkyl, aryl;
(b) 1-naphthyl, 2-naphthyl, 2-pyridyl, 3-pyridyl, 4-pyridyl, 2-thiophenyl, 3-thiophenyl, quinoline, isoquinoline, benzo[b]thiophene, 1,3-dihydrobenzo[c]thiophene, 1-dibenzofuran, 2-dibenzofuran, 3-dibenzofuran, 4-dibenzofuran; and;

Y-X is O=C-$CH_2$.

**7.** A pharmaceutical composition comprising

(a) a pharmacologically effective quantity of a compound of Formula I according to any of claims 1 to 6; and
(b) conventional pharmaceutically acceptable auxiliaries and/or carriers.

**8.** The composition according to Claim 7 wherein
R1 and R2 are independently selected from the group consisting of: hydrogen, $C_1$ to $C_4$ alkyl, $C_4$ to $C_7$ cycloalkyl, or, R1 and R2 together form a 5 or 6-membered ring which optionally may contain from 1 to 2 heteroatoms independently selected from the group consisting of nitrogen and/or oxygen atoms and which also may be substituted by optionally substituted aryl, optionally substituted heteroaryl, or arylenehalogenalkyl;
R3 to R6 are independently selected from the group consisting of: hydrogen, flourine, chlorine, bromine, methyl, ethyl, propyl, butyl, ethylene, propylene, methoxy, ethoxy, propoxy, ethinyl, propinyl, butinyl, $NSO_2CH_3$, $NH_2$, $NO_2$, aminomethyl, aminoethyl, aminopropyl, aminobutyl, aminodimethyl, aminodiethyl, aminodipropyl, aminodibutyl, cyano, oxyphenyl, oxybenzyl, oxyethylenephenyl, oxyphenylenemethyl, oxyphenylenemethoxy, acetyl, amidomethyl, amidoethyl, oxymethylenecarbonylmethyl, oxyethylenecarbonylmethyl, oxymethylenecarbonylethyl, oxyethylenecarbonylethyl, oxymethyleneoxymethyl, oxymethyleneoxyethyl, oxyethyleneoxymethyl, oxyethyleneoxyethyl, amidooxymethyl, and amidooxyethyl; and
Y-X is selected from the group consisting of: HC=CH, $CH_2$-$CH_2$, O=C-$CH_2$, and (HO)(H)C-$CH_2$.

**9.** The composition according to any of Claims 7 to 8 wherein
R1 and R2 are both H;
R3 to R6 are independently selected from the group consisting of: hydrogen, halogen and $C_1$ to $C_4$ alkoxy; and
Y-X is O=C-$CH_2$-

**10.** The composition according to any of Claims 7 to 9 wherein
R4 is selected from the group consisting of: hydrogen, chlorine, bromine, and methoxy; and
R5 is selected from the group consisting of: hydrogen and bromine.

**11.** The composition according to any of Claims 7 to 10 wherein
R1 and R2 are independently selected from the group consisting of: hydrogen, $C_1$ to $C_4$ alkyl, $C_4$ to $C_7$ cycloalkyl, or, R1 and R2 together form a 5 or 6-membered ring which optionally may contain from 1 to 2 heteroatoms independently selected from the group consisting of nitrogen and/or oxygen atoms and which also may be substituted by optionally substituted aryl, optionally substituted heteroaryl or arylenehalogenalkyl;
R3, R5 and R6 are independently selected from the group consisting of: hydrogen halogen; $C_1$ to $C_4$ alkyl; $C_2$ to $C_4$ alkenyl; $C_1$ to $C_4$ alkoxy; $C_1$ to $C_4$ alkoxy substituted with halogen provided that the alpha-carbon atom is substituted by no other halogen than fluorine if any; $C_2$ to $C_4$ alkinyl; $C_1$ to $C_4$ $NSO_2$alkyl; $NH_2$; $NO_2$; $C_1$, to $C_4$ aminoalkyl; $C_2$ to $C_8$ aminodialkyl; cyano; oxyaryl; oxyalkylenearyl; oxyarylenealkyl; oxyalkylenearylenealkoxy; $C_2$ to $C_8$ ester; $C_1$ to $C_8$ amido; $C_2$ to $C_8$ oxyalkylenecarbonylalkyl; $C_2$ to $C_8$ oxyalkyleneoxyalkyl; and; $C_1$ to $C_4$ amidooxyalkyl;
R4 is selected from the group consisting of:

(a) phenyl, optionally substituted with one to three substituents independently selected from the group consisting of: hydrogen, amino, halogen, $C_1$ to $C_4$ alkyl, $C_1$ to $C_4$ alkoxy, oxyaryl, $C_1$ to $C_4$ mercapto, C(O)H, trifluormethyl, -N-C(O)$C_{1-4}$alkyl, $C_2$ to $C_4$ alkenyl, C(O)$C_{1-4}$alkyl, and aryl;
(b) 1-naphthyl, 2-naphthyl, 2-pyridyl, 3-pyridyl, 4-pyridyl, 2-thiophenyl, 3-thiophenyl, quinoline, isoquinoline, benzo[b]thiophene, 1,3-dihydrobenzo[c]thiophene, 1-dibenzofuran, 2-dibenzofuran, 3-dibenzofuran, and, 4-dibenzofuran, each optionally substituted with one to three substituents selected from the group consisting of: hydrogen, amino, halogen, $C_1$ to $C_4$ alkyl, $C_1$ to $C_4$ alkoxy, oxyaryl, $C_1$ to $C_4$ mercapto, C(O)H, trifluormethyl, -N-C(O)$C_{1-4}$alkyl, $C_2$ to $C_4$ alkenyl, C(O)$C_{1-4}$alkyl, and aryl; and;

Y-X is selected from the group consisting of: HC=CH, $CH_2$-$CH_2$, O=C-$CH_2$, and (HO)(H)C-$CH_2$.

**12.** The composition according to Claim 11 wherein
R1, R2, R3, R5 and R6 are each hydrogen; and
R4 is selected from the group consisting of:

(a) phenyl, optionally substituted with one to three substituents independently selected from the group consisting of: hydrogen, amino, halogen, $C_1$ to $C_4$ alkyl, $C_1$ to $C_4$ alkoxy, oxyaryl, C1 to C4 mercapto, C(O)H, trifluormethyl,

- N-C(O)$C_{1-4}$alkyl, C2 to C4 alkenyl, C(O)$C_{1-4}$alkyl, aryl;

(b) 1-naphthyl, 2-naphthyl, 2-pyridyl, 3-pyridyl, 4-pyridyl, 2-thiophenyl, 3-thiophenyl, quinoline, isoquinoline, benzo[b]thiophene, 1,3-dihydrobenzo[c]thiophens, 1-dibenzofuran, 2-dibenzofuran, 3-dibenzofuran, 4-dibenzofuran; and;

Y-X is O=C-$CH_2$-

**13.** A process for the preparation of a compound of Formula I according to any of claims 1 to 6 **characterized in that**

(a) a benzopyrane compound of general Formula II,

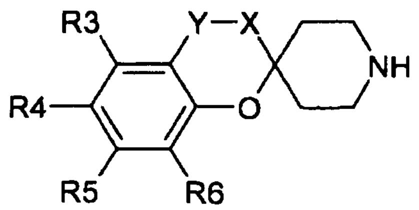

II

wherein R3 to R6, X and Y have the above meanings, is reacted with sulfamide to give a compound of formula I, or

(b) a benzopyrane of Formula II

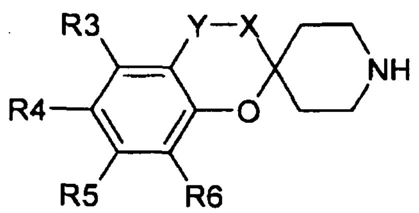

II

wherein R3 to R6, X and Y have the above meanings, is reacted with a compound of formula III

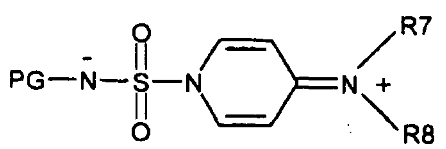

III

wherein R7 and R8 $C_1$ to $C_6$ alkyl and/or $C_3$ to $C_8$ cycloalkyl, wherein PG denotes a protecting group, preferably tert.-butyloxycarbonyl, and subsequently cleaving off the PG-group under suitable, preferably acidic, conditions from the obtained intermediate compound, to give a compound of formula I, or

(c) a benzopyrane of Formula II

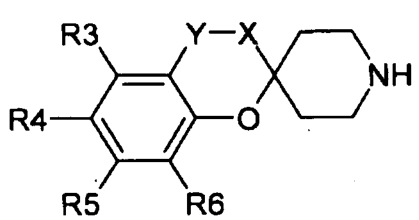

II

wherein R3 to R6, X and Y have the above meanings, is reacted with a sulfamoylchloride, which is protected

with a protecting group, preferably tert.-butyloxycarbonyl, of Formula IV,

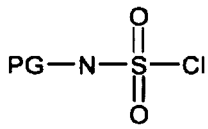

IV

and subsequently cleaving off the protecting group under suitable, preferably acidic, conditions from the obtained intermediate product, to give a compound of formula I, or,
(d) a benzopyrane of Formula II

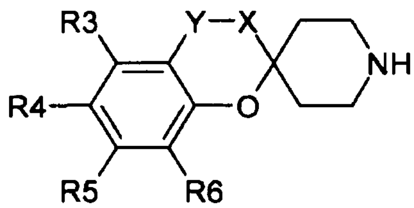

II

wherein R3 to R6, X and Y have the above meanings, is reacted with a sulfamoylchloride of Formula IVa,

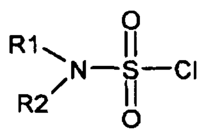

IVa

wherein R1 and R2 have the above meanings, to give a compound of formula I, or
(e) a benzopyrane of Formula I wherein at least one of R3 to R6 is bromo, chloro, or, iodo, preferably bromo or chloro, more preferably bromo, is reacted with a compound of formula IX

$$W\text{-}B(OH)_2 \qquad IX$$

wherein W is selected from the group consisting of: aryl, optionally substituted; heteroaryl; condensed aryl; and condensed heteraryl;
to give a compound of formula I wherein the bromo group in any of R3 to R6 of the starting material is now W;

and if desired converting resulting free bases of Formula I into their physiologically acceptable acid addition salts, or converting the acid addition salts of the compounds of Formula I into the free bases of Formula I.

**14.** The process according to Claim 13 wherein
R1 and R2 are independently selected from the group consisting of: hydrogen, $C_1$ to $C_4$ alkyl, $C_4$ to $C_7$ cycloalkyl, or, R1 and R2 together form a 5 or 6-membered ring which optionally may contain from 1 to 2 heteroatoms independently selected from the group consisting of nitrogen and/or oxygen atoms and which also may be substituted by optionally substituted aryl, optionally substituted heteroaryl, or arylenehalogenalkyl;
R3 to R6 are independently selected from the group consisting of: hydrogen, flourine, chlorine, bromine, methyl, ethyl, propyl, butyl, ethylene, propylene, methoxy, ethoxy, propoxy, ethinyl, propinyl, butinyl, $NSO_2CH_3$, $NH_2$, $NO_2$, aminomethyl, aminoethyl, aminopropyl, aminobutyl, aminodimethyl, aminodiethyl, aminodipropyl, aminodibutyl, cyano, oxyphenyl, oxybenzyl, oxyethylenephenyl, oxyphenylenemethyl, oxyphenylenemethoxy, acetyl, amidomethyl, amidoethyl, oxymethylenecarbonylmethyl, oxyethylenecarbonylmethyl, oxymethylenecarbonylethyl, oxyethylenecarbonylethyl, oxymethyleneoxymethyl, oxymethyleneoxyethyl, oxyethyleneoxymethyl, oxyethyleneoxyethyl, amidooxymethyl, and amidooxyethyl; and
Y-X is selected from the group consisting of: HC=CH, $CH_2$-$CH_2$, O=C-$CH_2$, and (HO)(H)C-$CH_2$.

**15.** The process according to any of Claims 13 to 14 wherein
R1 and R2 are both H;
R3 to R6 are independently selected from the group consisting of: hydrogen, halogen and $C_1$ to $C_4$ alkoxy; and
Y-X is O=C-$CH_2$.

**16.** The process according to any of Claims 13 to 15 wherein
R4 is selected from the group consisting of: hydrogen, chlorine, bromine, and methoxy; and
R5 is selected from the group consisting of: hydrogen and bromine.

**17.** The process according to any of Claims 13 to 16 wherein
R1 and R2 are independently selected from the group consisting of: hydrogen, $C_1$ to $C_4$ alkyl, $C_4$ to $C_7$ cycloalkyl, or, R1 and R2 together form a 5 or 6-membered ring which optionally may contain from 1 to 2 heteroatoms independently selected from the group consisting of nitrogen and/or oxygen atoms and which also may be substituted by optionally substituted aryl, optionally substituted heteroaryl or arylenehalogenalkyl;
R3, R5 and R6 are independently selected from the group consisting of: hydrogen; halogen; $C_1$ to $C_4$ alkyl; $C_2$ to $C_4$ alkenyl; $C_1$ to $C_4$ alkoxy; $C_1$ to $C_4$ alkoxy substituted with halogen provided that the alpha-carbon atom is substituted by no other halogen than fluorine if any; $C_2$ to $C_4$ alkinyl; $C_1$ to $C_4$ $NSO_2$alkyl; $NH_2$; $NO_2$; $C_1$ to $C_4$ aminoalkyl; $C_2$ to $C_8$ aminodialkyl; cyano; oxyaryl; oxyalkylenearyl; oxyarylenealkyl; oxyalkylenearylenealkoxy; $C_2$ to $C_8$ ester; $C_1$ to $C_8$ amido; $C_2$ to $C_8$ oxyalkylenecarbonylalkyl; $C_2$ to $C_8$ oxyalkyleneoxyalkyl; and; $C_1$ to $C_4$ amidooxyalkyl;
R4 is selected from the group consisting of:

(a) phenyl, optionally substituted with one to three substituents independently selected from the group consisting of: hydrogen, amino, halogen, $C_1$ to $C_4$ alkyl, $C_1$ to $C_4$ alkoxy, oxyaryl, $C_1$ to $C_4$ mercapto, C(O)H, trifluormethyl, -N-C(O)$C_{1-4}$alkyl, $C_2$ to $C_4$ alkenyl, C(O)$C_{1-4}$alkyl, and aryl;
(b) 1-naphthyl, 2-naphthyl, 2-pyridyl, 3-pyridyl, 4-pyridyl, 2-thiophenyl, 3-thiophenyl, quinoline, isoquinoline, benzo[b]thiophene, 1,3-dihydrobenzo[c]thiophene, 1-dibenzofuran, 2-dibenzofuran, 3-dibenzofuran, and, 4-dibenzofuran, each optionally substituted with one to three substituents selected from the group consisting of: hydrogen, amino, halogen, $C_1$ to $C_4$ alkyl, $C_1$ to $C_4$ alkoxy, oxyaryl, $C_1$ to $C_4$ mercapto, C(O)H, trifluormethyl, -N-C(O)$C_{1-4}$alkyl, $C_2$ to $C_4$ alkenyl, C(O)$C_{1-4}$alkyl, and aryl; and;

Y-X is selected from the group consisting of: HC=CH, $CH_2$-$CH_2$, O=C-$CH_2$, and (HO)(H)C-$CH_2$.

**18.** The process according to any of Claims 13 to 17 wherein
R1, R2, R3, R5 and R6 are each hydrogen; and
R4 is selected from the group consisting of:

(a) phenyl, optionally substituted with one to three substituents independently selected from the group consisting of: hydrogen, amino, halogen, $C_1$ to $C_4$ alkyl, $C_1$ to $C_4$ alkoxy, oxyaryl, C1 to C4 mercapto, C(O)H, trifluormethyl, - N-C(O)$C_{1-4}$alkyl, C2 to C4 alkenyl, C(O)$C_{1-4}$alkyl, aryl;
(b) 1-naphthyl, 2-naphthyl, 2-pyridyl, 3-pyridyl, 4-pyridyl, 2-thiophenyl, 3-thiophenyl, quinoline, isoquinoline, benzo[b]thiophene, 1,3-dihydrobenzo[c]thiophene, 1-dibenzofuran, 2-dibenzofuran, 3-dibenzofuran, 4-dibenzofuran; and;

Y-X is O=C-$CH_2$.

**19.** The use of a compound of Formula I according to any of claims 1 to 6 for the preparation of a medicament for the prophylaxis and/or treatment and/or prevention and/or inhibition of epilepsy, bipolar disorders, migraine, neuropathic pain, obesity, type II diabetes, metabolic syndrome, alcohol dependence, and/or cancer, and its concomitant and/or secondary diseases or conditions in mammals, preferably humans.

**20.** A compound of Formula I according to any of claims 1 to 6 for use in the treatment and/or for use in the prevention and/or for use in the inhibition of epilepsy, bipolar disorders, migraine, neuropathic pain, obesity, type II diabetes, metabolic syndrome, alcohol dependence, and/or cancer, and its concomitant and/or secondary diseases or conditions in mammals and humans.

**Patentansprüche**

1. Verbindungen der Formel I

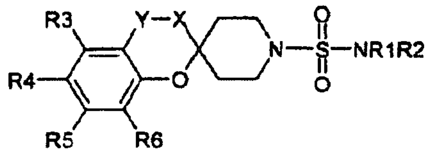

I

wobei
R1 und R2 unabhängig voneinander aus der aus Wasserstoff, $C_1$- bis $C_4$-Alkyl und $C_4$- bis $C_7$-Cycloalkyl bestehenden Gruppe ausgewählt sind; oder
R1 und R2 zusammen einen 5- oder 6-gliedrigen Ring bilden, der gegebenenfalls 1 oder 2 unabhängig voneinander aus der aus Stickstoff- und/oder Sauerstoffatomen bestehenden Gruppe ausgewählte Heteroatome enthalten kann und der außerdem durch gegebenenfalls substituiertes Aryl, gegebenenfalls substituiertes Heteroaryl oder Arylenhalogenalkyl substituiert sein kann;
R3 bis R6 unabhängig voneinander aus der aus Wasserstoff; Halogen; $C_1$- bis $C_4$-Alkyl; gegebenenfalls durch Aryl substituiertes $C_2$- bis $C_4$-Alkenyl; $C_1$- bis $C_4$-Alkoxy; $C_1$- bis $C_4$-Alkoxy, das durch Halogen substituiert ist, mit der Maßgabe, daß das alpha-Kohlenstoffatom durch kein anderes Halogen als Fluor substituiert ist, wenn überhaupt; $C_2$- bis $C_4$-Alkinyl; $C_1$- bis $C_4$-$NSO_2$-Alkyl; $NH_2$; $NO_2$; $C_1$- bis $C_4$-Aminoalkyl; $C_2$- bis $C_8$-Aminodialkyl; Cyano; Oxyaryl; Oxyalkylenaryl; Oxyarylenalkyl; Oxyalkylenarylenalkoxy; $C_2$- bis $C_8$-Ester; $C_1$- bis $C_8$-Amido; $C_2$- bis $C_8$-Oxyalkylencarbonylalkyl; $C_2$- bis $C_8$-Oxyalkylenoxyalkyl; $C_1$- bis $C_4$-Amidooxyalkyl; gegebenenfalls substituiertes Aryl; gegebenenfalls substituiertes Heteroaryl; gegebenenfalls substituiertes kondensiertes Aryl; gegebenenfalls substituiertes kondensiertes Heteroaryl bestehenden Gruppe ausgewählt sind; oder
R3 und R6 die gleiche Bedeutung wie oben haben und R4 und R5 zusammen einen 5- oder 6-gliedrigen Ring bilden, der gegebenenfalls 1 bis 3 unabhängig voneinander aus der aus Stickstoff, Sauerstoff und Schwefel bestehenden Gruppe ausgewählte Heteroatome enthalten kann und gegebenenfalls 1 oder 2 Doppelbindungen trägt und außerdem eine Carbonylgruppe enthalten kann und außerdem durch $C_1$- bis $C_4$-Alkyl, $C_1$- bis $C_4$-Halogenalkyl, gegebenenfalls substituiertes Aryl und/oder gegebenenfalls substituiertes Heteroaryl substituiert sein kann; oder
R5 und R6 die gleiche Bedeutung wie oben haben und R3 und R4 zusammen einen 5- oder 6-gliedrigen Ring bilden, der gegebenenfalls 1 bis 3 unabhängig voneinander aus der aus Stickstoff, Sauerstoff und Schwefel bestehenden Gruppe ausgewählte Heteroatome enthalten kann und gegebenenfalls 1 oder 2 Doppelbindungen trägt und außerdem eine Carbonylgruppe enthalten kann und außerdem durch $C_1$- bis $C_4$-Alkyl, $C_1$- bis $C_4$-Halogenalkyl, gegebenenfalls substituiertes Aryl und/oder gegebenenfalls substituiertes Heteroaryl substituiert sein kann; oder
R3 und R4 die gleiche Bedeutung wie oben haben und R5 und R6 zusammen einen 5- oder 6-gliedrigen Ring bilden, der gegebenenfalls 1 bis 3 unabhängig voneinander aus der aus Stickstoff, Sauerstoff und Schwefel bestehenden Gruppe ausgewählte Heteroatome enthalten kann und gegebenenfalls 1 oder 2 Doppelbindungen trägt und außerdem eine Carbonylgruppe enthalten kann und außerdem durch $C_1$- bis $C_4$-Alkyl, $C_1$- bis $C_4$-Halogenalkyl, gegebenenfalls substituiertes Aryl und/oder gegebenenfalls substituiertes Heteroaryl substituiert sein kann; oder
Y-X aus der aus HC=CH, $CH_2$-$CH_2$, O=C-$CH_2$ und (HO)(H)C-$CH_2$ bestehenden Gruppe ausgewählt ist;
und deren physiologisch unbedenkliche Säureadditionssalze.

2. Verbindungen nach Anspruch 1, wobei
R1 und R2 unabhängig voneinander aus der aus Wasserstoff, $C_1$- bis $C_4$-Alkyl und $C_4$- bis $C_7$-Cycloalkyl bestehenden Gruppe ausgewählt sind; oder
R1 und R2 zusammen einen 5- oder 6-gliedrigen Ring bilden, der gegebenenfalls 1 oder 2 unabhängig voneinander aus der aus Stickstoff- und/oder Sauerstoffatomen bestehenden Gruppe ausgewählte Heteroatome enthalten kann und der außerdem durch gegebenenfalls substituiertes Aryl, gegebenenfalls substituiertes Heteroaryl oder Arylenhalogenalkyl substituiert sein kann;
R3 bis R6 unabhängig voneinander aus der aus Wasserstoff, Fluor, Chlor, Brom, Methyl, Ethyl, Propyl, Butyl, Ethylen, Propylen, Methoxy, Ethoxy, Propoxy, Ethinyl, Propinyl, Butinyl, $NSO_2CH_3$, $NH_2$, $NO_2$, Aminomethyl, Aminoethyl, Aminopropyl, Aminobutyl, Aminodimethyl, Aminodiethyl, Aminodipropyl, Aminodibutyl, Cyano, Oxyphenyl, Oxybenzyl, Oxyethylenphenyl, Oxyphenylenmethyl, Oxyphenylenmethoxy, Acetyl, Amidomethyl, Amidoethyl, Oxymethylencarbonylmethyl, Oxyethylencarbonylmethyl, Oxymethylencarbonylethyl, Oxyethylencarbonylethyl, Oxymethylenoxymethyl, Oxymethylenoxyethyl, Oxyethylenoxymethyl, Oxyethylenoxyethyl, Amidooxymethyl und

Amidooxyethyl bestehenden Gruppe ausgewählt sind; und
Y-X aus der aus HC=CH, $CH_2$-$CH_2$, O=C-$CH_2$ und (HO)(H)C-CH2 bestehenden Gruppe ausgewählt ist.

**3.** Verbindungen nach Anspruch 1 oder 2, wobei R1 und R2 beide für H stehen;
R3 bis R6 unabhängig voneinander aus der aus Wasserstoff, Halogen und $C_1$- bis $C_4$-Alkoxy bestehenden Gruppe ausgewählt sind; und
Y-X für O=C-$CH_2$ steht.

**4.** Verbindungen nach einem der Ansprüche 1 bis 3, wobei
R4 aus der aus Wasserstoff, Chlor, Brom und Methoxy bestehenden Gruppe ausgewählt ist; und
R5 aus der aus Wasserstoff und Brom bestehenden Gruppe ausgewählt ist.

**5.** Verbindungen nach Anspruch 1, wobei
R1 und R2 unabhängig voneinander aus der aus Wasserstoff, $C_1$- bis $C_4$-Alkyl und $C_4$- bis $C_7$-Cycloalkyl bestehenden Gruppe ausgewählt sind, oder
R1 und R2 zusammen einen 5- oder 6-gliedrigen Ring bilden, der gegebenenfalls 1 bis 2 unabhängig voneinander aus der aus Stickstoff- und/oder Sauerstoffatomen bestehenden Gruppe ausgewählte Heteroatome enthalten kann und der außerdem durch gegebenenfalls substituiertes Aryl, gegebenenfalls substituiertes Heteroaryl oder Arylenhalogenalkyl substituiert sein kann;
R3, R5 und R6 unabhängig voneinander aus der aus Wasserstoff; Halogen; $C_1$- bis $C_4$-Alkyl; $C_2$- bis $C_4$-Alkenyl; $C_1$- bis $C_4$-Alkoxy; $C_1$- bis $C_4$-Alkoxy, das durch Halogen substituiert ist, mit der Maßgabe, daß das alpha-Kohlenstoffatom durch kein anderes Halogen als Fluor substituiert ist, wenn überhaupt; $C_2$- bis $C_4$-Alkinyl; $C_1$- bis $C_4$-$NSO_2$-Alkyl; $NH_2$; $NO_2$; $C_1$- bis $C_4$-Aminoalkyl; $C_2$- bis $C_8$-Aminodialkyl; Cyano; Oxyaryl; Oxyalkylenaryl; Oxyarylenalkyl; Oxyalkylenarylenalkoxy; $C_2$- bis $C_8$-Ester; $C_1$- bis $C_8$-Amido; $C_2$- bis $C_8$-Oxyalkylencarbonylalkyl; $C_2$- bis $C_8$-Oxyalkylenoxyalkyl und $C_1$- bis $C_4$-Amidooxyalkyl;
R4 aus der aus:

(a) Phenyl, welches gegebenenfalls durch einen bis drei unabhängig voneinander aus der aus Wasserstoff, Amino, Halogen, $C_1$- bis $C_4$-Alkyl, $C_1$- bis $C_4$-Alkoxy, Oxyaryl, $C_1$- bis $C_4$-Mercapto, C(O)H, Trifluormethyl, -N-C (0) -$C_{1-4}$-Alkyl, $C_2$- bis
$C_4$-Alkenyl, C(O)-$C_{1-4}$-Alkyl und Aryl bestehenden Gruppe ausgewählte Substituenten substituiert ist;
(b) 1-Naphtyl, 2-Naphtyl, 2-Pyridyl, 3-Pyridyl, 4-Pyridyl, 2-Thiophenyl, 3-Thiophenyl, Chinolin, Isochinolin, Benzo [b]thiophen, 1,3-Dihydrobenzo[c]thiophen, 1-Dibenzofuran, 2-Dibenzofuran, 3-Dibenzofuran und 4-Dibenzofuran, welche jeweils gegebenenfalls durch einen bis drei aus der aus Wasserstoff, Amino, Halogen, $C_1$- bis $C_4$-Alkyl, $C_1$- bis $C_4$-Alkoxy, Oxyaryl, $C_1$- bis $C_4$-Mercapto, C(O)H, Trifluormethyl, -N-C(O)-$C_{1-4}$-Alkyl, $C_2$- bis $C_4$-Alkenyl, C(O)-$C_{1-4}$-Alkyl und Aryl bestehenden Gruppe ausgewählte Substituenten substituiert sind;

bestehenden Gruppe ausgewählt ist; und
Y-X aus der aus HC=CH, $CH_2$-$CH_2$, O=C-$CH_2$ und (HO)(H)C-$CH_2$ bestehenden Gruppe ausgewählt ist.

**6.** Verbindungen nach Anspruch 5, wobei
R1, R2, R3, R5 und R6 jeweils für Wasserstoff stehen; und
R4 aus der aus:

(a) Phenyl, welches gegebenenfalls durch einen bis drei unabhängig voneinander aus der aus Wasserstoff, Amino, Halogen, $C_1$- bis $C_4$-Alkyl, $C_1$- bis $C_4$-Alkoxy, Oxyaryl, $C_1$- bis $C_4$-Mercapto, C(O)H, Trifluormethyl, -N-C(O)-$C_{1-4}$-Alkyl, $C_2$- bis $C_4$-Alkenyl, C(O)-$C_{1-4}$-Alkyl und Aryl bestehenden Gruppe ausgewählte Substituenten substituiert ist;
(b) 1-Naphtyl, 2-Naphtyl, 2-Pyridyl, 3-Pyridyl, 4-Pyridyl, 2-Thiophenyl, 3-Thiophenyl, Chinolin, Isochinolin, Benzo [b]thiophen, 1,3-Dihydrobenzo[c]thiophen, 1-Dibenzofuran, 2-Dibenzofuran, 3-Dibenzofuran und 4-Dibenzofuran;

bestehenden Gruppe ausgewählt ist; und
Y-X für O=C-$CH_2$ steht.

**7.** Pharmazeutische Zusammensetzung, enthaltend

(a)eine pharmakologisch wirksame Menge einer Verbindung der Formel I nach einem der Ansprüche 1 bis 6; und

(b)herkömmliche pharmazeutisch unbedenkliche Hilfsstoffe und/oder Träger.

**8.** Zusammensetzung nach Anspruch 7, wobei
R1 und R2 unabhängig voneinander aus der aus Wasserstoff, $C_1$- bis $C_4$-Alkyl und $C_4$- bis $C_7$-Cycloalkyl bestehenden Gruppe ausgewählt sind; oder
R1 und R2 zusammen einen 5- oder 6-gliedrigen Ring bilden, der gegebenenfalls 1 oder 2 unabhängig voneinander aus der aus Stickstoff- und/oder Sauerstoffatomen bestehenden Gruppe ausgewählte Heteroatome enthalten kann und der außerdem durch gegebenenfalls substituiertes Aryl, gegebenenfalls substituiertes Heteroaryl oder Arylenhalogenalkyl substituiert sein kann;
R3 bis R6 unabhängig voneinander aus der aus Wasserstoff, Fluor, Chlor, Brom, Methyl, Ethyl, Propyl, Butyl, Ethylen, Propylen, Methoxy, Ethoxy, Propoxy, Ethinyl, Propinyl, Butinyl, $NSO_2CH_3$, $NH_2$, $NO_2$, Aminomethyl, Aminoethyl, Aminopropyl, Aminobutyl, Aminodimethyl, Aminodiethyl, Aminodipropyl, Aminodibutyl, Cyano, Oxyphenyl, Oxybenzyl, Oxyethylenphenyl, Oxyphenylenmethyl, Oxyphenylenmethoxy, Acetyl, Amidomethyl, Amidoethyl, Oxymethylencarbonylmethyl, Oxyethylencarbonylmethyl, Oxymethylencarbonylethyl, Oxyethylencarbonylethyl, Oxymethylenoxymethyl, Oxymethylenoxyethyl, Oxyethylenoxymethyl, Oxyethylenoxyethyl, Amidooxymethyl und Amidooxyethyl; bestehenden Gruppe ausgewählt sind; und
Y-X aus der aus HC=CH, $CH_2$-$CH_2$, O=C-$CH_2$ und (HO)(H)C-$CH_2$ bestehenden Gruppe ausgewählt ist.

**9.** Zusammensetzung nach Anspruch 7 oder 8, wobei
R1 und R2 beide für H stehen;
R3 bis R6 unabhängig voneinander aus der aus Wasserstoff, Halogen und $C_1$- bis $C_4$-Alkoxy bestehenden Gruppe ausgewählt sind; und
Y-X für O=C-$CH_2$ steht.

**10.** Zusammensetzung nach einem der Ansprüche 7 bis 9, wobei
R4 aus der aus Wasserstoff, Chlor, Brom und Methoxy bestehenden Gruppe ausgewählt ist; und
R5 aus der aus Wasserstoff und Brom bestehenden Gruppe ausgewählt ist.

**11.** Zusammensetzung nach einem der Ansprüche 7 bis 10, wobei
R1 und R2 unabhängig voneinander aus der aus Wasserstoff, $C_1$- bis $C_4$-Alkyl und $C_4$- bis $C_7$-Cycloalkyl bestehenden Gruppe ausgewählt sind, oder
R1 und R2 zusammen einen 5- oder 6-gliedrigen Ring bilden, der gegebenenfalls 1 bis 2 unabhängig voneinander aus der aus Stickstoff- und/oder Sauerstoffatomen bestehenden Gruppe ausgewählte Heteroatome enthalten kann und der außerdem durch gegebenenfalls substituiertes Aryl, gegebenenfalls substituiertes Heteroaryl oder Arylenhalogenalkyl substituiert sein kann;
R3, R5 und R6 unabhängig voneinander aus der aus Wasserstoff; Halogen; $C_1$- bis $C_4$-Alkyl; $C_2$- bis $C_4$-Alkenyl; $C_1$- bis $C_4$-Alkoxy; $C_1$- bis $C_4$-Alkoxy, das durch Halogen substituiert ist, mit der Maßgabe, daß das alpha-Kohlenstoffatom durch kein anderes Halogen als Fluor substituiert ist, wenn überhaupt; $C_2$- bis $C_4$-Alkinyl; $C_1$- bis $C_4$-$NSO_2$-Alkyl; $NH_2$; $NO_2$; $C_1$- bis $C_4$-Aminoalkyl; $C_2$- bis $C_8$-Aminodialkyl; Cyano; Oxyaryl; Oxyalkylenaryl; Oxyarylenalkyl; Oxyalkylenarylenalkoxy; $C_2$- bis $C_8$-Ester; $C_1$- bis $C_8$-Amido; $C_2$- bis $C_8$-Oxyalkylencarbonylalkyl; $C_2$- bis $C_8$-Oxyalkylenoxyalkyl und $C_1$- bis $C_4$-Amidooxyalkyl;
R4 aus der aus:

(a) Phenyl, welches gegebenenfalls durch einen bis drei unabhängig voneinander aus der aus Wasserstoff, Amino, Halogen, $C_1$- bis Aralkyl, $C_1$- bis $C_4$-Alkoxy, Oxyaryl, $C_1$- bis $C_4$-Mercapto, C(O)H, Trifluormethyl, -N-C(O)-$C_{1-4}$-Alkyl, $C_2$- bis $C_4$-Alkenyl, C(O)-$C_{1-4}$-Alkyl und Aryl bestehenden Gruppe ausgewählte Substituenten substituiert ist;
(b) 1-Naphtyl, 2-Naphtyl, 2-Pyridyl, 3-Pyridyl, 4-Pyridyl, 2-Thiophenyl, 3-Thiophenyl, Chinolin, Isochinolin, Benzo[b]thiophen, 1,3-Dihydrobenzo[c]thiophen, 1-Dibenzofuran, 2-Dibenzofuran, 3-Dibenzofuran und 4-Dibenzofuran, welche jeweils gegebenenfalls durch einen bis drei aus der aus Wasserstoff, Amino, Halogen, $C_1$- bis $C_4$-Alkyl, $C_1$- bis $C_4$-Alkoxy, Oxyaryl, $C_1$- bis $C_4$-Mercapto, C(O)H, Trifluormethyl, -N-C(O)-$C_{1-4}$-Alkyl, $C_2$- bis $C_4$-Alkenyl, C(O)-$C_{1-4}$-Alkyl und Aryl bestehenden Gruppe ausgewählte Substituenten substituiert sind;

bestehenden Gruppe ausgewählt ist; und
Y-X aus der aus HC=CH, $CH_2$-$CH_2$, O=C-$CH_2$ und (HO)(H)C-$CH_2$ bestehenden Gruppe ausgewählt ist.

**12.** Zusammensetzung nach Anspruch 11, wobei
R1, R2, R3, R5 und R6 jeweils für Wasserstoff stehen; und

R4 aus der aus:

(a) Phenyl, welches gegebenenfalls durch einen bis drei unabhängig voneinander aus der aus Wasserstoff, Amino, Halogen, $C_1$- bis $C_4$-Alkyl, $C_1$- bis $C_4$-Alkoxy, Oxyaryl, $C_1$- bis $C_4$-Mercapto, C(O)H, Trifluormethyl, -N-C(O)-$C_{1-4}$-Alkyl, $C_2$- bis $C_4$-Alkenyl, C(O)-$C_{1-4}$-Alkyl und Aryl bestehenden Gruppe ausgewählte Substituenten substituiert ist;
(b) 1-Naphtyl, 2-Naphtyl, 2-Pyridyl, 3-Pyridyl, 4-Pyridyl, 2-Thiophenyl, 3-Thiophenyl, Chinolin, Isochinolin, Benzo[b]thiophen, 1,3-Dihydrobenzo[c]thiophen, 1-Dibenzofuran, 2-Dibenzofuran, 3-Dibenzofuran und 4-Dibenzofuran;

bestehenden Gruppe ausgewählt ist; und
Y-X für O=C-$CH_2$ steht.

**13.** Verfahren zur Herstellung einer Verbindung der Formel I nach einem der Ansprüche 1 bis 6, **dadurch gekennzeichnet, daß** man

(a) eine Benzopyranverbindung der allgemeinen Formel II

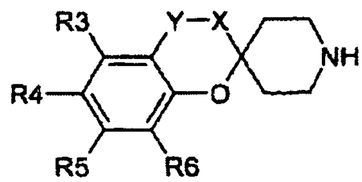 II

,

in welcher R3 bis R6, X und Y die obigen Bedeutungen haben, mit einem Sulfamid zu einer Verbindung der Formel I umsetzt, oder
(b) ein Benzopyran der Formel II

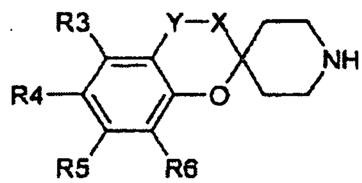 II

,

in welcher R3 bis R6, X und Y die obigen Bedeutungen haben, mit einer Verbindung der Formel III

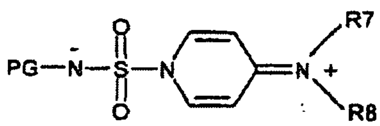 III

,

in welcher R7 und R8 für $C_1$- bis $C_6$-Alkyl und/oder $C_3$- bis $C_8$-Cycloalkyl stehen, wobei PG für eine Schutzgruppe, vorzugsweise tert.-Butyloxycarbonyl, steht, umsetzt und anschließend die PG-Gruppe unter geeigneten, vorzugsweise sauren, Bedingungen von dem erhaltenen Zwischenprodukt abspaltet, wodurch man eine Verbindungen der Formel I erhält, oder
(c) ein Benzopyran der Formel II

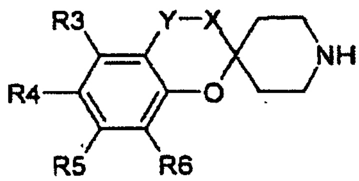 II

,

in welcher R3 bis R6, X und Y die obigen Bedeutungen haben, mit einem durch eine Schutzgruppe, vorzugsweise tert.-Butyloxycarbonyl, geschütztem Sulfamoylchlorid der Formel IV

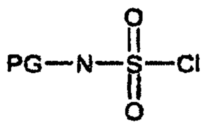 IV

umsetzt und anschließend die Schutzgruppe unter geeigneten, vorzugsweise sauren, Bedingungen von dem erhaltenen Zwischenprodukt abspaltet, wodurch man eine Verbindung der Formel I erhält, oder
(d) ein Benzopyran der Formel II

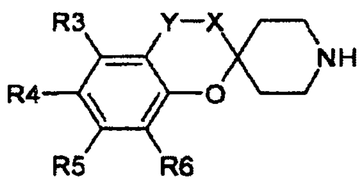 II

,

in welcher R3 bis R6, X und Y die obigen Bedeutungen haben, mit einem Sulfamoylchlorid der Formel IVa

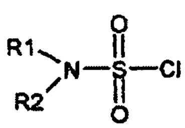 IVa

,

in welcher R1 und R2 die obigen Bedeutungen haben, zu einer Verbindungen der Formel I umsetzt, oder
(e) ein Benzopyran der Formel I, in welcher wenigstens einer der Reste R3 bis R6 für Brom, Chlor oder Iod, vorzugsweise Brom oder Chlor, besonders bevorzugt Brom, steht, mit einer Verbindungen der Formel IX

$$W\text{-}B(OH)_2 \qquad IX,$$

in welcher W aus der aus gegebenenfalls substituierten Aryl; Heteroaryl; kondensiertem Aryl und kondensiertem Heteroaryl bestehenden Gruppe ausgewählt ist;
zu einer Verbindung der Formel I, in welcher die Brom-Gruppe in einem der Reste R3 bis R6 des Ausgangsmaterials jetzt für W steht, umsetzt;

und, falls gewünscht, erhaltene freie Basen der Formel I in ihre physiologisch unbedenklichen Säureadditionssalze umwandelt oder die Säureadditionssalze der Verbindungen der Formel I in die freien Basen der Formel I umwandelt.

**14.** Verfahren nach Anspruch 13, wobei
R1 und R2 unabhängig voneinander aus der aus Wasserstoff, $C_1$- bis $C_4$-Alkyl und $C_4$- bis $C_7$-Cycloalkyl bestehenden Gruppe ausgewählt sind; oder
R1 und R2 zusammen einen 5- oder 6-gliedrigen Ring bilden, der gegebenenfalls 1 oder 2 unabhängig voneinander aus der aus Stickstoff- und/oder Sauerstoffatomen bestehenden Gruppe ausgewählte Heteroatome enthalten kann und der außerdem durch gegebenenfalls substituiertes Aryl, gegebenenfalls substituiertes Heteroaryl oder Arylenhalogenalkyl substituiert sein kann;
R3 bis R6 unabhängig voneinander aus der aus Wasserstoff, Fluor, Chlor, Brom, Methyl, Ethyl, Propyl, Butyl, Ethylen, Propylen, Methoxy, Ethoxy, Propoxy, Ethinyl, Propinyl, Butinyl, $NSO_2CH_3$, $NH_2$, $NO_2$, Aminomethyl, Aminoethyl, Aminopropyl, Aminobutyl, Aminodimethyl, Aminodiethyl, Aminodipropyl, Aminodibutyl, Cyano, Oxyphenyl, Oxybenzyl, Oxyethylenphenyl, Oxyphenylenmethyl, Oxyphenylenmethoxy, Acetyl, Amidomethyl, Amidoethyl, Oxymethylencarbonylmethyl, Oxyethylencarbonylmethyl, Oxymethylencarbonylethyl, Oxyethylencarbonylethyl, Oxymethylenoxymethyl, Oxymethylenoxyethyl, Oxyethylenoxymethyl, Oxyethylenoxyethyl, Amidooxymethyl und Amidooxyethyl bestehenden Gruppe ausgewählt sind; und
Y-X aus der aus HC=CH, $CH_2$-$CH_2$, O=C-$CH_2$ und (HO)(H)C-$CH_2$ bestehenden Gruppe ausgewählt ist.

**15.** Verfahren nach Anspruch 13 oder 14, wobei
R1 und R2 beide für H stehen;
R3 bis R6 unabhängig voneinander aus der aus Wasserstoff, Halogen und $C_1$- bis $C_4$-Alkoxy bestehenden Gruppe ausgewählt sind; und
Y-X für O=C-$CH_2$ steht.

**16.** Verfahren nach einem der Ansprüche 13 bis 15, wobei
R4 aus der aus Wasserstoff, Chlor, Brom und Methoxy bestehenden Gruppe ausgewählt ist; und
R5 aus der aus Wasserstoff und Brom bestehenden Gruppe ausgewählt ist.

**17.** Verfahren nach einem der Ansprüche 13 bis 16, wobei
R1 und R2 unabhängig voneinander aus der aus Wasserstoff, $C_1$- bis $C_4$-Alkyl und $C_4$- bis $C_7$-Cycloalkyl bestehenden Gruppe ausgewählt sind, oder
R1 und R2 zusammen einen 5- oder 6-gliedrigen Ring bilden, der gegebenenfalls 1 bis 2 unabhängig voneinander aus der aus Stickstoff- und/oder Sauerstoffatomen bestehenden Gruppe ausgewählte Heteroatome enthalten kann und der außerdem durch gegebenenfalls substituiertes Aryl, gegebenenfalls substituiertes Heteroaryl oder Arylenhalogenalkyl substituiert sein kann;
R3, R5 und R6 unabhängig voneinander aus der aus Wasserstoff; Halogen; $C_1$- bis $C_4$-Alkyl; $C_2$- bis $C_4$-Alkenyl; $C_1$- bis $C_4$-Alkoxy; $C_1$- bis $C_4$-Alkoxy, das durch Halogen substituiert ist, mit der Maßgabe, daß das alpha-Kohlenstoffatom durch kein anderes Halogen als Fluor substituiert ist, wenn überhaupt; $C_2$- bis $C_4$-Alkinyl; $C_1$- bis $C_4$-$NSO_2$-Alkyl; $NH_2$; $NO_2$; $C_1$- bis $C_4$-Aminoalkyl; $C_2$- bis $C_8$-Aminodialkyl; Cyano; Oxyaryl; Oxyalkylenaryl; Oxyarylenalkyl; Oxyalkylenarylenalkoxy; $C_2$- bis $C_8$-Ester; $C_1$- bis $C_8$-Amido; $C_2$- bis $C_8$-Oxyalkylen-carbonylalkyl; $C_2$- bis $C_8$-Oxyalkylenoxyalkyl und $C_1$- bis $C_4$-Amidooxyalkyl;
R4 aus der aus:

(a) Phenyl, welches gegebenenfalls durch einen bis drei unabhängig voneinander aus der aus Wasserstoff, Amino, Halogen, $C_1$- bis $C_4$-Alkyl, $C_1$- bis $C_4$-Alkoxy, Oxyaryl, $C_1$- bis $C_4$-Mercapto, C(O)H, Trifluormethyl, -N-C(O)-$C_{1-4}$-Alkyl, $C_2$- bis $C_4$-Alkenyl, C(O)-$C_{1-4}$-Alkyl und Aryl bestehenden Gruppe ausgewählte Substituenten substituiert ist;
(b) 1-Naphtyl, 2-Naphtyl, 2-Pyridyl, 3-Pyridyl, 4-Pyridyl, 2-Thiophenyl, 3-Thiophenyl, Chinolin, Isochinolin, Benzo[b]thiophen, 1,3-Dihydrobenzo[c]thiophen, 1-Dibenzofuran, 2-Dibenzofuran, 3-Dibenzofuran und 4-Dibenzofuran, welche jeweils gegebenenfalls durch einen bis drei aus der aus Wasserstoff, Amino, Halogen, $C_1$- bis $C_4$-Alkyl, $C_1$- bis $C_4$-Alkoxy, Oxyaryl, $C_1$- bis $C_4$-Mercapto, C(O)H, Trifluormethyl, -N-C(O)-$C_{1-4}$-Alkyl, $C_2$- bis $C_4$-Alkenyl, C(O)-$C_{1-4}$-Alkyl und Aryl bestehenden Gruppe ausgewählte Substituenten substituiert sind;

bestehenden Gruppe ausgewählt ist; und
Y-X aus der aus HC=CH, $CH_2$-$CH_2$, O=C-$CH_2$ und (HO)(H)C-$CH_2$ bestehenden Gruppe ausgewählt ist.

**18.** Verfahren nach einem der Ansprüche 13 bis 17, wobei
R1, R2, R3, R5 und R6 jeweils für Wasserstoff stehen; und
R4 aus der aus:

(a) Phenyl, welches gegebenenfalls durch einen bis drei unabhängig voneinander aus der aus Wasserstoff, Amino, Halogen, $C_1$- bis $C_4$-Alkyl, $C_1$- bis $C_4$-Alkoxy, Oxyaryl, $C_1$- bis $C_4$-Mercapto, C(O)H, Trifluormethyl, -N-C(O)-$C_{1-4}$-Alkyl, $C_2$- bis $C_4$-Alkenyl, C(O)-$C_{1-4}$-Alkyl und Aryl bestehenden Gruppe ausgewählte Substituenten substituiert ist;
(b) 1-Naphtyl, 2-Naphtyl, 2-Pyridyl, 3-Pyridyl, 4-Pyridyl, 2-Thiophenyl, 3-Thiophenyl, Chinolin, Isochinolin, Benzo [b]thiophen, 1,3-Dihydrobenzo[c]thiophen, 1-Dibenzofuran, 2-Dibenzofuran, 3-Dibenzofuran und 4-Dibenzofuran;

bestehenden Gruppe ausgewählt ist; und
Y-X für O=C-$CH_2$ steht.

**19.** Verwendung einer Verbindung der Formel I nach einem der Ansprüche 1 bis 6 zur Herstellung eines Medikaments zur Prophylaxe und/oder Behandlung und/oder Prävention und/oder Inhibierung von Epilepsie, bipolaren Störungen, Migräne, neuropathischen Schmerzen, Obesitas, Typ-II-Diabetes, metabolischem Syndrom, Alkoholabhängigkeit und/oder Krebs und damit einhergehenden Begleit- und/oder Sekundärkrankheiten bzw. -leiden in Säugetieren, vorzugsweise Menschen.

**20.** Verbindungen der Formel I nach einem der Ansprüche 1 bis 6 zur Verwendung bei der Behandlung und/oder zur Verwendung bei der Prävention und/oder zur Verwendung bei der Inhibierung von Epilepsie, bipolaren Störungen, Migräne, neuropathischen Schmerzen, Obesitas, Typ-II-Diabetes, metabolischem Syndrom, Alkoholabhängigkeit und/oder Krebs und damit einhergehenden Begleit- und/oder Sekundärkrankheiten bzw. -leiden in Säugetieren und Menschen.

## Revendications

**1.** Composé de formule I

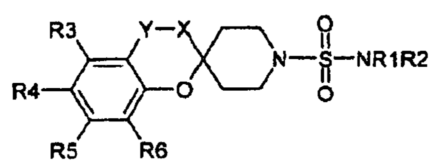

I

dans laquelle
R1 et R2 sont choisis indépendamment dans le groupe constitué par : hydrogène, alkyle en $C_1$ à $C_4$, cycloalkyle en $C_4$ à $C_7$ ; ou
R1 et R2 forment ensemble un cycle à 5 ou 6 éléments qui peut éventuellement contenir 1 à 2 hétéroatomes choisis indépendamment dans le groupe constitué par les atomes d'azote et/ou d'oxygène et qui peut également être substitué par un aryle éventuellement substitué,
un hétéroaryle éventuellement substitué ou un arylènehalogénoalkyle ;
R3 à R6 sont choisis indépendamment dans le groupe constitué par : hydrogène ; halogène ; alkyle en $C_1$ à $C_4$ ; alcényle en $C_2$ à $C_4$ éventuellement substitué avec un aryle ; alcoxy en $C_1$ à $C_4$ ; alcoxy en $C_1$ à $C_4$ substitué avec un halogène à condition que l'atome de carbone alpha ne soit substitué par aucun autre halogène que le fluor le cas échéant ; alcynyle en $C_2$ à $C_4$ ; $NSO_2$alkyle en $C_1$ à $C_4$ ; $NH_2$ ; $NO_2$ ; aminoalkyle en $C_1$ à $C_4$ ; aminodialkyle en $C_2$ à $C_8$; cyano ; oxyaryle ; oxyalkylènearyle ; oxyarylènealkyle ; oxyalkylènearylènealcoxy ; ester en $C_2$ à $C_8$ ; amido en $C_1$ à $C_8$ ; oxyalkylènecarbonylalkyle en $C_2$ à $C_8$ ; oxyalkylèneoxyalkyle en $C_2$ à $C_8$ ; amidooxyalkyle en $C_1$ à $C_4$ ; aryle éventuellement substitué ; hétéroaryle éventuellement substitué ; aryle condensé éventuellement substitué ; hétéroaryle condensé éventuellement substitué ; ou ;
R3 et R6 ont la même signification que ci-dessus et R4 et R5 forment ensemble un cycle à 5 ou 6 éléments qui peut éventuellement contenir 1 à 3 hétéroatomes choisis indépendamment dans le groupe constitué par : l'azote, l'oxygène et le soufre, et qui peut éventuellement porter 1 ou 2 doubles liaisons, et qui peut également contenir un groupe carbonyle, et qui peut également être substitué par un alkyle en $C_1$ à $C_4$, un halogénoalkyle en $C_1$ à $C_4$, un aryle éventuellement substitué et/ou un hétéroaryle éventuellement substitué ; ou ;
R5 et R6 ont la même signification que ci-dessus et R3 et R4 forment ensemble un cycle à 5 ou 6 éléments qui peut éventuellement contenir 1 à 3 hétéroatomes choisis indépendamment dans le groupe constitué par : l'azote,

l'oxygène et le soufre, et qui peut éventuellement porter 1 ou 2 doubles liaisons, et qui peut également contenir un groupe carbonyle, et qui peut également être substitué par un alkyle en $C_1$ à $C_4$, un halogénoalkyle en $C_1$ à $C_4$, un aryle éventuellement substitué et/ou un hétéroaryle éventuellement substitué ; ou ;
R3 et R4 ont la même signification que ci-dessus et R5 et R6 forment ensemble un cycle à 5 ou 6 éléments qui peut éventuellement contenir 1 à 3 hétéroatomes choisis indépendamment dans le groupe constitué par : l'azote, l'oxygène et le soufre, et qui peut éventuellement porter 1 ou 2 doubles liaisons, et qui peut également contenir un groupe carbonyle, et qui peut également être substitué par un alkyle en $C_1$ à $C_4$, un halogénoalkyle en $C_1$ à $C_4$, un aryle éventuellement substitué et/ou un hétéroaryle éventuellement substitué ;
Y-X est choisi dans le groupe constitué par : HC=CH, $CH_2$-$CH_2$, O=C-$CH_2$ et (HO)(H)C-$CH_2$ ;
et son sel d'addition acide physiologiquement acceptable.

**2.** Composé selon la revendication 1, dans lequel
R1 et R2 sont choisis indépendamment dans le groupe constitué par : hydrogène, alkyle en $C_1$ à $C_4$, cycloalkyle en $C_4$ à $C_7$, ou
R1 et R2 forment ensemble un cycle à 5 ou 6 éléments qui peut éventuellement contenir 1 à 2 hétéroatomes choisis indépendamment dans le groupe constitué par les atomes d'azote et/ou d'oxygène et qui peut également être substitué par un aryle éventuellement substitué, un hétéroaryle éventuellement substitué ou un arylènehalogénoalkyle ;
R3 à R6 sont choisis indépendamment dans le groupe constitué par : hydrogène, fluor, chlore, brome, méthyle, éthyle, propyle, butyle, éthylène, propylène, méthoxy, éthoxy, propoxy, éthynyle, propynyle, butynyle, $NSO_2CH_3$, $NH_2$, $NO_2$, aminométhyle, aminoéthyle, aminopropyle, aminobutyle, aminodiméthyle, aminodiéthyle, aminodipropyle, aminodibutyle, cyano, oxyphényle, oxybenzyle, oxyéthylènephényle, oxyphénylèneméthyle, oxyphénylèneméthoxy, acétyle, amidométhyle, amidoéthyle, oxyméthylènecarbonylméthyle, oxyéthylènecarbonylméthyle, oxyméthylènecarbonyléthyle, oxyéthylènecarbonyléthyle, oxyméthylèneoxyméthyle, oxyméthylèneoxyéthyle, oxyéthylèneoxyméthyle, oxyéthylèneoxyéthyle, amidooxyméthyle et amidooxyéthyle ; et
Y-X est choisi dans le groupe constitué par : HC=CH, $CH_2$-$CH_2$, O=C-$CH_2$ et (HO)(H)C-$CH_2$.

**3.** Composé selon l'une quelconque des revendications 1 ou 2, dans lequel
R1 et R2 sont tous les deux H ;
R3 à R6 sont choisis indépendamment dans le groupe constitué par : hydrogène, halogène et alcoxy en $C_1$ à $C_4$ ; et
Y-X est O=C-$CH_2$.

**4.** Composé selon l'une quelconque des revendications 1 à 3, dans lequel
R4 est choisi dans le groupe constitué par : hydrogène, chlore, brome et méthoxy ; et
R5 est choisi dans le groupe constitué par : hydrogène et brome.

**5.** Composé selon la revendication 1, dans lequel
R1 et R2 sont choisis indépendamment dans le groupe constitué par : hydrogène, alkyle en $C_1$ à $C_4$, cycloalkyle en $C_4$ à $C_7$, ou
R1 et R2 forment ensemble un cycle à 5 ou 6 éléments qui peut éventuellement contenir 1 à 2 hétéroatomes choisis indépendamment dans le groupe constitué par les atomes d'azote et/ou d'oxygène et qui peut également être substitué par un aryle éventuellement substitué, un hétéroaryle éventuellement substitué ou un arylènehalogénoalkyle ;
R3, R5 et R6 sont choisis indépendamment dans le groupe constitué par : hydrogène ; halogène ; alkyle en $C_1$ à $C_4$ ; alcényle en $C_2$ à $C_4$ ; alcoxy en $C_1$ à $C_4$ ; alcoxy en $C_1$ à $C_4$ substitué avec un halogène à condition que l'atome de carbone alpha ne soit substitué par aucun autre halogène que le fluor le cas échéant ; alcynyle en $C_2$ à $C_4$ ; $NSO_2$alkyle en $C_1$ à $C_4$ ; $NH_2$ ; $NO_2$ ; aminoalkyle en $C_1$ à $C_4$ ; aminodialkyle en $C_2$ à $C_8$; cyano ; oxyaryle ; oxyalkylènearyle ; oxyarylènealkyle ; oxyalkylènearylènealcoxy ; ester en $C_2$ à $C_8$ ; amido en $C_1$ à $C_8$ ; oxyalkylènecarbonylalkyle en $C_2$ à $C_8$ ; oxyalkylèneoxyalkyle en $C_2$ à $C_8$ ; et amidooxyalkyle en $C_1$ à $C_4$ ;
R4 est choisi dans le groupe constitué par :

(a) phényle, éventuellement substitué avec un à trois substituants choisis indépendamment dans le groupe constitué par : hydrogène, amino, halogène, alkyle en $C_1$ à $C_4$, alcoxy en $C_1$ à $C_4$, oxyaryle, mercapto en $C_1$ à $C_4$, C(O)H, trifluorométhyle, -N-C(O)alkyle en $C_{1-4}$, alcényle en $C_2$ à $C_4$, C(O)alkyle en $C_{1-4}$ et aryle ;
(b) 1-naphtyle, 2-naphtyle, 2-pyridyle, 3-pyridyle, 4-pyridyle, 2-thiophényle, 3-thiophényle, quinoline, isoquinoline, benzo[b]thiophène, 1,3-dihydrobenzo[c]thiophène, 1-dibenzofurane, 2-dibenzofurane, 3-dibenzofurane et 4-dibenzofurane, chacun éventuellement substitué avec un à trois substituants choisis dans le groupe constitué par : hydrogène, amino, halogène, alkyle en $C_1$ à $C_4$, alcoxy en $C_1$ à $C_4$, oxyaryle, mercapto en $C_1$ à $C_4$, C(O)

H, trifluorométhyle, -N-C(O)alkyle en $C_{1-4}$, alcényle en $C_2$ à $C_4$, C(O)alkyle en $C_{1-4}$ et aryle ; et ;

Y-X est choisi dans le groupe constitué par : HC=CH, $CH_2$-$CH_2$, O=C-$CH_2$ et (HO)(H)C-$CH_2$.

**6.** Composé selon la revendication 5, dans lequel
R1, R2, R3, R5 et R6 sont chacun l'hydrogène ; et
R4 est choisi dans le groupe constitué par :

(a) phényle, éventuellement substitué avec un à trois substituants choisis indépendamment dans le groupe constitué par : hydrogène, amino, halogène, alkyle en $C_1$ à $C_4$, alcoxy en $C_1$ à $C_4$, oxyaryle, mercapto en $C_1$ à $C_4$, C(O)H, trifluorométhyle, -N-C(O)alkyle en $C_{1-4}$, alcényle en $C_2$ à $C_4$, C(O)alkyle en $C_{1-4}$ et aryle ;
(b) 1-naphtyle, 2-naphtyle, 2-pyridyle, 3-pyridyle, 4-pyridyle, 2-thiophényle, 3-thiophényle, quinoline, isoquinoline, benzo[b]thiophène, 1,3-dihydrobenzo[c]thiophène, 1-dibenzofurane, 2-dibenzofurane, 3-dibenzofurane et 4-dibenzofurane ; et ;

Y-X est O=C-$CH_2$.

**7.** Composition pharmaceutique comprenant

(a) une quantité pharmacologiquement efficace d'un composé de formule I selon l'une quelconque des revendications 1 à 6 ; et
(b) des auxiliaires et/ou véhicules pharmaceutiquement acceptables classiques.

**8.** Composition selon la revendication 7, dans laquelle
R1 et R2 sont choisis indépendamment dans le groupe constitué par : hydrogène, alkyle en $C_1$ à $C_4$, cycloalkyle en $C_4$ à $C_7$ ; ou
R1 et R2 forment ensemble un cycle à 5 ou 6 éléments qui peut éventuellement contenir 1 à 2 hétéroatomes choisis indépendamment dans le groupe constitué par les atomes d'azote et/ou d'oxygène et qui peut également être substitué par un aryle éventuellement substitué, un hétéroaryle éventuellement substitué ou un arylènehalogénoalkyle ;
R3 à R6 sont choisis indépendamment dans le groupe constitué par : hydrogène, fluor, chlore, brome, méthyle, éthyle, propyle, butyle, éthylène, propylène, méthoxy, éthoxy, propoxy, éthynyle, propynyle, butynyle, $NSO_2CH_3$, $NH_2$, $NO_2$, aminométhyle, aminoéthyle, aminopropyle, aminobutyle, aminodiméthyle, aminodiéthyle, aminodipropyle, aminodibutyle, cyano, oxyphényle, oxybenzyle, oxyéthylènephényle, oxyphénylèneméthyle, oxyphénylèneméthoxy, acétyle, amidométhyle, amidoéthyle, oxyméthylènecarbonylméthyle, oxyéthylènecarbonylméthyle, oxyméthylènecarbonyléthyle, oxyéthylènecarbonyléthyle, oxyméthylèneoxyméthyle, oxyméthylèneoxyéthyle, oxyéthylèneoxyméthyle, oxyéthylèneoxyéthyle, amidooxyméthyle et amidooxyéthyle ; et
Y-X est choisi dans le groupe constitué par : HC=CH, $CH_2$-$CH_2$, O=C-$CH_2$ et (HO)(H)C-$CH_2$.

**9.** Composition selon l'une quelconque des revendications 7 à 8, dans laquelle
R1 et R2 sont tous les deux H ;
R3 à R6 sont choisis indépendamment dans le groupe constitué par : hydrogène, halogène et alcoxy en $C_1$ à $C_4$ ; et
Y-X est O=C-$CH_2$.

**10.** Composition selon l'une quelconque des revendications 7 à 9, dans laquelle
R4 est choisi dans le groupe constitué par : hydrogène, chlore, brome et méthoxy ; et
R5 est choisi dans le groupe constitué par : hydrogène et brome.

**11.** Composition selon l'une quelconque des revendications 7 à 10, dans laquelle
R1 et R2 sont choisis indépendamment dans le groupe constitué par : hydrogène, alkyle en $C_1$ à $C_4$, cycloalkyle en $C_4$ à $C_7$, ou
R1 et R2 forment ensemble un cycle à 5 ou 6 éléments qui peut éventuellement contenir 1 à 2 hétéroatomes choisis indépendamment dans le groupe constitué par les atomes d'azote et/ou d'oxygène et qui peut également être substitué par un aryle éventuellement substitué, un hétéroaryle éventuellement substitué ou un arylènehalogénoalkyle ;
R3, R5 et R6 sont choisis indépendamment dans le groupe constitué par : hydrogène ; halogène ; alkyle en $C_1$ à $C_4$ ; alcényle en $C_2$ à $C_4$ ; alcoxy en $C_1$ à $C_4$ ; alcoxy en $C_1$ à $C_4$ substitué avec un halogène à condition que l'atome de carbone alpha ne soit substitué par aucun autre halogène que le fluor le cas échéant ; alcynyle en $C_2$ à $C_4$ ;

$NSO_2$alkyle en $C_1$ à $C_4$ ; $NH_2$ ; $NO_2$ ; aminoalkyle en $C_1$ à $C_4$ ; aminodialkyle en $C_2$ à $C_8$; cyano ; oxyaryle ; oxyalkylènearyle ; oxyarylènealkyle ; oxyalkylènearylènealcoxy ; ester en $C_2$ à $C_8$ ; amido en $C_1$ à $C_8$ ; oxyalkylènecarbonylalkyle en $C_2$ à $C_8$ ; oxyalkylèneoxyalkyle en $C_2$ à $C_8$ et amidooxyalkyle en $C_1$ à $C_4$ ;
R4 est choisi dans le groupe constitué par :

(a) phényle, éventuellement substitué avec un à trois substituants choisis indépendamment dans le groupe constitué par : hydrogène, amino, halogène, alkyle en $C_1$ à $C_4$, alcoxy en $C_1$ à $C_4$, oxyaryle, mercapto en $C_1$ à $C_4$, C(O)H, trifluorométhyle, -N-C(O)alkyle en $C_{1-4}$, alcényle en $C_2$ à $C_4$, C(O)alkyle en $C_{1-4}$ et aryle ;
(b) 1-naphtyle, 2-naphtyle, 2-pyridyle, 3-pyridyle, 4-pyridyle, 2-thiophényle, 3-thiophényle, quinoline, isoquinoline, benzo[b]thiophène, 1,3-dihydrobenzo[c]thiophène, 1-dibenzofurane, 2-dibenzofurane, 3-dibenzofurane et 4-dibenzofurane, chacun éventuellement substitué avec un à trois substituants choisis dans le groupe constitué par : hydrogène, amino, halogène, alkyle en $C_1$ à $C_4$, alcoxy en $C_1$ à $C_4$, oxyaryle, mercapto en $C_1$ à $C_4$, C(O)H, trifluorométhyle, -N-C(O)alkyle en $C_{1-4}$, alcényle en $C_2$ à $C_4$, C(O)alkyle en $C_{1-4}$ et aryle ; et ;

Y-X est choisi dans le groupe constitué par : HC=CH, $CH_2$-$CH_2$, O=C-$CH_2$ et (HO)(H)C-$CH_2$.

**12.** Composition selon la revendication 11, dans laquelle
R1, R2, R3, R5 et R6 sont chacun l'hydrogène ; et
R4 est choisi dans le groupe constitué par :

(a) phényle, éventuellement substitué avec un à trois substituants choisis indépendamment dans le groupe constitué par : hydrogène, amino, halogène, alkyle en $C_1$ à $C_4$, alcoxy en $C_1$ à $C_4$, oxyaryle, mercapto en $C_1$ à $C_4$, C(O)H, trifluorométhyle, -N-C(O)alkyle en $C_{1-4}$, alcényle en $C_2$ à $C_4$, C(O)alkyle en $C_{1-4}$ et aryle ;
(b) 1-naphtyle, 2-naphtyle, 2-pyridyle, 3-pyridyle, 4-pyridyle, 2-thiophényle, 3-thiophényle, quinoline, isoquinoline, benzo[b]thiophène, 1,3-dihydrobenzo[c]thiophène, 1-dibenzofurane, 2-dibenzofurane, 3-dibenzofurane et 4-dibenzofurane ; et ;

Y-X est O=C-$CH_2$.

**13.** Procédé de préparation d'un composé de formule I selon l'une quelconque des revendications 1 à 6, **caractérisé en ce que**

(a) un composé de benzopyrane de formule générale II

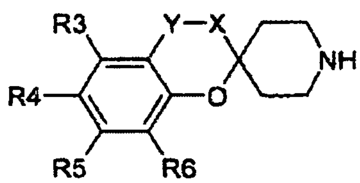

II

dans laquelle R3 à R6, X et Y ont les significations ci-dessus, est mis en réaction avec du sulfamide pour obtenir un composé de formule I, ou
(b) un benzopyrane de formule II

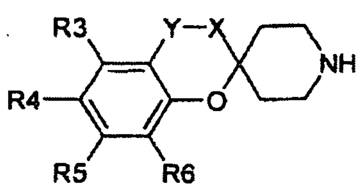

II

dans laquelle R3 à R6, X et Y ont les significations ci-dessus, est mis en réaction avec un composé de ) formule III

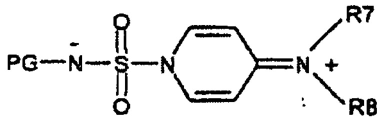

III

dans laquelle R7 et R8 représentent un alkyle en $C_1$ à $C_6$ et/ou un cycloalkyle en $C_3$ à $C_8$, PG représente un groupe protecteur, de préférence un tert.-butyloxycarbonyle, puis le groupe PG est clivé dans des conditions appropriées, de préférence acides, du composé intermédiaire obtenu pour obtenir un composé de formule I, ou
(c) un benzopyrane de formule II

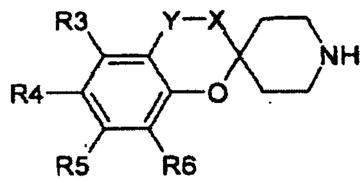

II

dans laquelle R3 à R6, X et Y ont les significations ci-dessus, est mis en réaction avec un chlorure de sulfamoyle, qui est protégé avec un groupe protecteur, de préférence le tert.-butyloxycarbonyle, de formule IV

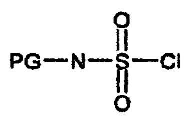

IV

puis le groupe protecteur est clivé dans des conditions appropriées, de préférence acides, du produit intermédiaire obtenu pour obtenir un composé de formule I, ou
(d) un benzopyrane de formule II

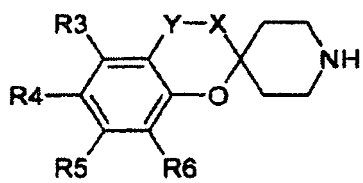

II

dans laquelle R3 à R6, X et Y ont les significations ci-dessus, est mis en réaction avec un chlorure de sulfamoyle de formule IVa,

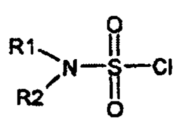

IVa

dans laquelle R1 et R2 ont les significations ci-dessus, pour obtenir un composé de formule I, ou
(e) un benzopyrane de formule I, dans lequel au moins un radical parmi R3 à R6 est bromo, chloro ou iodo, de préférence bromo ou chloro, de manière davantage préférée bromo, est mis en réaction avec un composé de formule IX

$W\text{-}B(OH)_2$ IX

dans laquelle W est choisi dans le groupe constitué par : aryle éventuellement substitué ; hétéroaryle ; aryle condensé ; et hétéroaryle condensé ;
pour obtenir un composé de formule I dans lequel le groupe bromo d'un radical quelconque parmi R3 à R6 de la matière première est maintenant W ;
et, si on le souhaite, les bases libres résultantes de formule I sont transformées en leurs sels d'addition acide physiologiquement acceptables, ou les sels d'addition acide des composés de formule I sont transformés en bases libres de formule I.

**14.** Procédé selon la revendication 13, dans lequel R1 et R2 sont choisis indépendamment dans le groupe constitué par : hydrogène, alkyle en $C_1$ à $C_4$, cycloalkyle en $C_4$ à $C_7$ ; ou
R1 et R2 forment ensemble un cycle à 5 ou 6 éléments qui peut éventuellement contenir 1 à 2 hétéroatomes choisis indépendamment dans le groupe constitué par les atomes d'azote et/ou d'oxygène et qui peut également être substitué par un aryle éventuellement substitué, un hétéroaryle éventuellement substitué ou un arylènehalogénoalkyle ;
R3 à R6 sont choisis indépendamment dans le groupe constitué par : hydrogène, fluor, chlore, brome, méthyle, éthyle, propyle, butyle, éthylène, propylène, méthoxy, éthoxy, propoxy, éthynyle, propynyle, butynyle, $NSO_2CH_3$, $NH_2$, $NO_2$, aminométhyle, aminoéthyle, aminopropyle, aminobutyle, aminodiméthyle, aminodiéthyle, aminodipropyle, aminodibutyle, cyano, oxyphényle, oxybenzyle, oxyéthylènephényle, oxyphénylèneméthyle, oxyphénylèneméthoxy, acétyle, amidométhyle, amidoéthyle, oxyméthylènecarbonylméthyle, oxyéthylènecarbonylméthyle, oxyméthylènecarbonyléthyle, oxyéthylènecarbonyléthyle, oxyméthylèneoxyméthyle, oxyméthylèneoxyéthyle, oxyéthylèneoxyméthyle, oxyéthylèneoxyéthyle, amidooxyméthyle et amidooxyéthyle ; et
Y-X est choisi dans le groupe constitué par : HC=CH, $CH_2$-$CH_2$, O=C-$CH_2$ et (HO)(H)C-$CH_2$.

**15.** Procédé selon l'une quelconque des revendications 13 à 14, dans lequel
R1 et R2 sont tous les deux H ;
R3 à R6 sont choisis indépendamment dans le groupe constitué par : hydrogène, halogène et alcoxy en $C_1$ à $C_4$ ; et
Y-X est O=C-$CH_2$.

**16.** Procédé selon l'une quelconque des revendications 13 à 15, dans lequel
R4 est choisi dans le groupe constitué par : hydrogène, chlore, brome et méthoxy ; et
R5 est choisi dans le groupe constitué par : hydrogène et brome.

**17.** Procédé selon l'une quelconque des revendications 13 à 16, dans lequel
R1 et R2 sont choisis indépendamment dans le groupe constitué par : hydrogène, alkyle en $C_1$ à $C_4$, cycloalkyle en $C_4$ à $C_7$, ou
R1 et R2 forment ensemble un cycle à 5 ou 6 éléments qui peut éventuellement contenir 1 à 2 hétéroatomes choisis indépendamment dans le groupe constitué par les atomes d'azote et/ou d'oxygène et qui peut également être substitué par un aryle éventuellement substitué, un hétéroaryle éventuellement substitué ou un arylènehalogénoalkyle ;
R3, R5 et R6 sont choisis indépendamment dans le groupe constitué par : hydrogène ; halogène ; alkyle en $C_1$ à $C_4$ ; alcényle en $C_2$ à $C_4$ ; alcoxy en $C_1$ à $C_4$ ; alcoxy en $C_1$ à $C_4$ substitué avec un halogène à condition que l'atome de carbone alpha ne soit substitué par aucun autre halogène que le fluor le cas échéant ; alcynyle en $C_2$ à $C_4$ ; $NSO_2$alkyle en $C_1$ à $C_4$ ; $NH_2$ ; $NO_2$ ; aminoalkyle en $C_1$ à $C_4$ ; aminodialkyle en $C_2$ à $C_8$; cyano ; oxyaryle ; oxyalkylènearyle ; oxyarylènealkyle ; oxyalkylènearylènealcoxy ; ester en $C_2$ à $C_8$ ; amido en $C_1$ à $C_8$ ; oxyalkylènecarbonylalkyle en $C_2$ à $C_8$ ; oxyalkylèneoxyalkyle en $C_2$ à $C_8$ et amidooxyalkyle en $C_1$ à $C_4$ ;
R4 est choisi dans le groupe constitué par :

(a) phényle, éventuellement substitué avec un à trois substituants choisis indépendamment dans le groupe constitué par : hydrogène, amino, halogène, alkyle en $C_1$ à $C_4$, alcoxy en $C_1$ à $C_4$, oxyaryle, mercapto en $C_1$ à $C_4$, C(O)H, trifluorométhyle, -N-C(O)alkyle en $C_{1-4}$, alcényle en $C_2$ à $C_4$, C(O)alkyle en $C_{1-4}$ et aryle ;
(b) 1-naphtyle, 2-naphtyle, 2-pyridyle, 3-pyridyle, 4-pyridyle, 2-thiophényle, 3-thiophényle, quinoline, isoquinoline, benzo[b]thiophène, 1,3-dihydrobenzo[c]thiophène, 1-dibenzofurane, 2-dibenzofurane, 3-dibenzofurane et 4-dibenzofurane, chacun éventuellement substitué avec un à trois substituants choisis dans le groupe constitué par : hydrogène, amino, halogène, alkyle en $C_1$ à $C_4$, alcoxy en $C_1$ à $C_4$, oxyaryle, mercapto en $C_1$ à $C_4$, C(O)H, trifluorométhyle, -N-C(O)alkyle en $C_{1-4}$, alcényle en $C_2$ à $C_4$, C(O)alkyle en $C_{1-4}$ et aryle ; et ;

Y-X est choisi dans le groupe constitué par : HC=CH, $CH_2$-$CH_2$, O=C-$CH_2$ et (HO)(H)C-$CH_2$.

**18.** Procédé selon l'une quelconque des revendications 13 à 17, dans lequel
R1, R2, R3, R5 et R6 sont chacun l'hydrogène ; et
R4 est choisi dans le groupe constitué par :

(a) phényle, éventuellement substitué avec un à trois substituants choisis indépendamment dans le groupe constitué par : hydrogène, amino, halogène, alkyle en $C_1$ à $C_4$, alcoxy en $C_1$ à $C_4$, oxyaryle, mercapto en $C_1$ à $C_4$, C(O)H, trifluorométhyle, -N-C(O)alkyle en $C_{1-4}$, alcényle en $C_2$ à $C_4$, C(O)alkyle en $C_{1-4}$ et aryle ;
(b) 1-naphtyle, 2-naphtyle, 2-pyridyle, 3-pyridyle, 4-pyridyle, 2-thiophényle, 3-thiophényle, quinoline, isoquinoline, benzo[b]thiophène, 1,3-dihydrobenzo[c]thiophène, 1-dibenzofurane, 2-dibenzofurane, 3-dibenzofurane et 4-dibenzofurane ; et ;

Y-X est O=C-$CH_2$.

**19.** Utilisation d'un composé de formule I selon l'une quelconque des revendications 1 à 6 pour la préparation d'un médicament pour la prophylaxie et/ou le traitement et/ou la prévention et/ou l'inhibition de l'épilepsie, des troubles bipolaires, de la migraine, de la douleur neuropathique, de l'obésité, du diabète de type II, du syndrome métabolique, de la dépendance à l'alcool et/ou du cancer, et ses maladies ou troubles concomitants et/ou secondaires chez les mammifères, de préférence les hommes.

**20.** Composé de formule I selon l'une quelconque des revendications 1 à 6 pour une utilisation dans le traitement et/ou pour une utilisation dans la prévention et/ou pour une utilisation dans l'inhibition de l'épilepsie, des troubles bipolaires, de la migraine, de la douleur neuropathique, de l'obésité, du diabète de type II, du syndrome métabolique, de la dépendance à l'alcool et/ou du cancer, et ses maladies ou troubles concomitants et/ou secondaires chez les mammifères et les hommes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0431943 A
- WO 0207821 A
- WO 9530642 A
- JP 2005119987 A
- WO 2004092179 A
- WO 9808871 A
- WO 9726265 A
- WO 9903861 A
- WO 9741097 A


### Non-patent literature cited in the description


- **Elliott et al.** *J. Med. Chem.,* 1992, vol. 35, 3973-3976
- **Nerenberg et al.** *Bioorganic & Medical Chemistry Letters,* 1999, vol. 9, 291-294
- **Yamato.** *J. Med. Chem.,* 1981, vol. 24, 194-198
- **Fletcher et al.** *J. Med. Chem.,* 2002, vol. 45, 492-503
- **Buchwald et al.** *Angew. Chem. Int. Ed.,* 2004, vol. 43, 1871-1876